# EUROPÄISCHE PATENTSCHRIFT

(11) **EP 2 785 390 B1**
(45) Veröffentlichungstag und Bekanntmachung des Hinweises auf die Patenterteilung: **07.10.2020**
(21) Anmeldenummer: 12798158.7
(22) Anmeldetag: 28.11.2012
(51) Int. Cl.: A61M 1/00

(54) **WUNDPFLEGEVORRICHTUNG ZUR BEHANDLUNG VON WUNDEN MITTELS ATMOSPHÄRISCHEM UNTERDRUCK**
WOUND CARE DEVICE FOR TREATING WOUNDS BY MEANS OF SUBATMOSPHERIC PRESSURE
DISPOSITIF DE SOIN DE PLAIES DESTINÉ À TRAITER DES PLAIES AU MOYEN D'UNE DÉPRESSION ATMOSPHÉRIQUE

(30) Priorität: 28.11.2011 DE 102011055782
(43) Veröffentlichungstag der Anmeldung: 08.10.2014
(73) Patentinhaber: BSN medical GmbH, 20253 Hamburg (DE)
(72) Erfinder: RIESINGER, Birgit, 48149 Münster (DE)
(74) Vertreter: FARAGO Patentanwälte
(86) Internationale Anmeldenummer: PCT/EP2012/004916
(87) Internationale Veröffentlichungsnummer: WO 2013/079196

(56) Entgegenhaltungen:
- WO-A1-2011/135284
- WO-A1-2013/076450
- DE-U1-202005 019 670
- US-A1- 2007 078 366
- US-A1- 2007 265 586
- US-A1- 2010 100 075
- US-A1- 2010 298 793
- US-A1- 2011 112 492
- US-B1- 7 198 046

## Beschreibung

Die Erfindung betrifft eine Wundpflegevorrichtung zur Behandlung von Wunden mittels atmosphärischem Unterdruck.

Herkömmliche Systeme und Vorrichtungen zur Behandlung von Wunden mittels atmosphärischem Unterdruck bestehen aus einer gasdichten Wundabdeckung, einem Drainageschlauch, einer extern angeordnete Vakuumpumpe sowie einem Sammelgefäß für die Aufnahme von abgeführten Exsudaten
Solche Vorrichtungen sind beispielsweise im Patent US7198046 beschrieben. Nachteilig für den Patienten ist es dabei, das besagte Vorrichtungen zur Behandlung von Wunden mittels atmosphärischem Unterdruck in aller Regel nicht mobil ausgebildet sind, d.h. eine Therapie ist nur lokal möglich, beispielsweise über eine am Krankenbett angeordnete Pumpe. Dies bindet den Patienten an das Bett und raubt ihm daher Lebensqualität. Alternativ muß der Patient dann, wenn er sich bewegen möchte, die Pumpe entfernen und so die Therapie unterbrechen.

Aus dem Stand der Technik sind auch weitere Wundpflegevorrichtungen bekannt.

Zum Beispiel offenbart Dokument US2007/0265586 A1 eine Vorrichtung zur Wundtherapie aufweisend eine Mikrovakuumpumpe, ein Wundabdeckungselement und einen beispielsweise aus einem superabsorbierenden Polymer bestehenden Flüssigkeitsaufnehmer.

Dokument US2011/0112492 A1 offenbart eine Wundpflegevorrichtung aufweisend eine Mikropumpe, eine Wundabdeckung und einen Absorptionskörper aus einem superabsorbierenden Polymer.

Dokument WO2011/135284 A1 offenbart eine Wundpflegevorrichtung mit einer externen Vakuumpumpe, einer Wundabdeckung und einem superabsorbierenden Zellulosefasern aufweisenden Absorptionskörper.

Aufgabe der vorliegenden Erfindung ist es, die Vakuum-Wundversorgung für den Patienten komfortabler zu machen. Weitere Aufgabe der vorliegenden Erfindung ist es, eine kontinuierliche Vakuum-Wundversorgung mit weniger Unterbrechungen für den Patienten zu gewährleisten.

Diese Aufgaben werden mit den Merkmalen der vorgelegten Ansprüche gelöst.

### AUSFÜHRLICHE BESCHREIBUNG DER ERFINDUNG

Erfindungsgemäß ist eine Wundpflegevorrichtung zur Behandlung von Wunden mittels atmosphärischem Unterdruck im Wundbereich vorgesehen, wie im Anspruch 1 definiert.

In Ergänzung hierzu kann vorgesehen sein, dass die Vorrichtung ferner wenigstens ein Wundexsudate aufnehmendes Reservoir aufweist, beispielsweise einen Kanister.

Ferner ist eine Einrichtung zur Erzeugung von atmosphärischem Unterdruck offenbart, die bevorzugt ausgewählt ist aus der Gruppe enthaltend
a) Elektrisch betriebene Vakuumpumpe
b) Handbetriebene Unterdruckquelle, und/oder
c) Evakuiertes Vakuumgefäß.

Die besagte Vakuumpumpe kann eine solitäre Pumpe sein, sie kann jedoch auch Bestandteil eines zentralisierten Saugsystems sein, wie es in Kliniken häufig zum Einsatz kommt. Dabei werden in den Patientenzimmern wandinstallierte Vakuumanschlüsse bereitgestellt, an welche die offenbarten Drainagevorrichtungen zur Wundbehandlung angeschlossen werden können. In diesem Fall kann besagte Vakuumpumpe eine Vielzahl von offenbarten Drainagevorrichtungen zur Wundbehandlung unter Unterdruck setzen.

Die erfindungsgemäße Wundpflegevorrichtung beinhaltet eine Mikropumpe, deren Ausmaße und/oder deren Gewicht so beschaffen ist, dass sie ohne weiteres an einem Wundabdeckungselement der genannten Art angebracht werden kann, ohne dass dies dem Patienten lästig fällt oder ihn in irgendeiner Weise stört.

Diese Pumpe kann beispielsweise vom Typ Piezo- oder Membranpumpe sein. Besonders bevorzugt sind Piezopumpen, also Pumpen, bei denen die Pumpleistung durch ein piezoelektrisches Element bewerkstelligt wird. Diese Pumpen weisen eine ausreichend hohe Pumpleistung bei geringen Ausmaßen, geringem Betriebsgeräusch und niedriger Energieaufnahme auf. Ebenso kann es sich um eine in Mikrosystemtechnik gefertigte Treibmittelvakuumpumpe handeln. Geeignete Pumpen dieses Typs werden beispielsweise von Schwarzer Precision, KNF oder Bartels Mikrotechnik hergestellt.

Bevorzugt ist eine solche Pumpe ferner mit einem Rückschlagventil ausgestattet. Auf diese Weise kann die Pumpe im Intervallmodus betrieben werden oder lediglich zum initialen Aufbau des Unterdrucks bzw. zum Erhalt des Unterdrucks eingeschaltet werden, ohne dass in den Betriebspausen Leckagen entstehen, die den aufgebauten Unterdruck wieder abbauen.

Bevorzugt ist die besagte Pumpe imstande, Unterdrücke von -20 bis -200 mm Hg zu ziehen. Ganz besonders bevorzugt ist vorgesehen, dass die Pumpe Unterdrücke von -20, -30, -40, - 50, -60, -70, -80, -85, -90, -100, -110, -120, -130, -140, -150, -160, -170, -180, -190 oder - 200 mm Hg zieht.

Die hier offenbarten und diskutierten genannten Druckwerte beziehen sich immer relativ zum Normaldruck von 1 bar = 750 mm Hg.

Besonders bevorzugt sind dabei Unterdrücke im Bereich zwischen -60 und -110 mm Hg, also -60, -61, -62, -63, -64, -65, -66, -67, -68, -69, -70, -71, -72,-73, -74, -75, -76, -77, -78, -79, - 80, -81, -82, -83, -84, -85, -86, -87, -88, -89, -90, -91, -92, -93, -94, -95, -96, -97, -98, -99, - 100, -101, -102, -103, -104, -105, -106, -107, -108, -109 und/oder -110 mm Hg.

Insbesondere durch die Synergie zwischen superabsorbierenden Polymeren und atmosphärische Unterdruck, die beide einen Sog und damit eine mobiliserende Wirkung auf die in der Wunde befindlichen Exsudate haben, kann ggf. ein geringerer Unterdruck gewählt werden als dies im Stand der Technik üblich, da - wie beschrieben - der atmosphärische Unterdruck durch die Superabsorbierenden Polymere komplimentiert wird. Auf diese Weise läßt sich das Betriebsgeräusch und die damit verbundenen Vibrationen erniedrigen, die Batteriegröße verkleinern und/oder die Batterielaufzeit verlängern.

Daher ist in einer alternativen Ausführungsform vorgesehen, dass die Unterdrücke im Bereich zwischen -20 und -80 mm Hg liegen, also -20, -21, -22, -23, -24, -25, -26, -27, -28, -29, -30, - 31, -32, -33, -34, -35, -36, -37, -38, -39, -40, -41, -42, -43, -44, -45, -46, -47, -48, -49, -50, - 51, -52, -53, -54, -55, -56, -57, -58, -59, -60, -61, -62, -63, -64, -65, -66, -67, -68, -69, -70, - 71, -72, -73, -74, -75, -76, -77, -78, -79 und/oder -80 mm Hg.

In einer weiteren alternativen Ausführungsform vorgesehen, dass die Unterdrücke im Bereich zwischen -100 und -140 mm Hg liegen, also -100, -101, -102, -103, -104, -105, -106, -107, - 108, -109, -110, -111, -112, -113, -114, -115, -116, -117, -118, -119, -120, -121, -122, -123, - 124, -125, -126, -127, -128, -129, -130, -131, -132, -133, -134, -135, -136, -137, -138, -139, und/oder -140 mm Hg.

In einer weiteren alternativen Ausführungsform vorgesehen, dass die Unterdrücke im Bereich zwischen -110 und -135 mm Hg liegen, also -110, -111, -112, -113, -114, -115, -116, -117,-118, -119, -120, -121, -122, -123, -124, -125, -126, -127, -128, -129 und/oder -130 mm Hg.

In einer weiteren alternativen Ausführungsform vorgesehen, dass die Unterdrücke im Bereich zwischen -120 und -125 mm Hg liegen, also -120, -121, -122, -123, -124 und/oder -120 mm Hg.

Bevorzugt ist die Pumpe so gewählt bzw. ausgestaltet, dass sie in der Lage ist, Flüssigkeiten zu befördern.

Bevorzugt überschreitet das Betriebsgeräusch der Pumpe einen Schalldruckpegel von 75 dB nicht. Besonders bevorzugt wird ein Schalldruckpegel von 73 dB, 70 dB, 68 dB, 65 dB, 63 dB, 60 dB, 58 dB, 55 dB, 53 dB, 50 dB, 48 dB, 45 dB, 42 dB, 40 dB, 38 dB, 35 dB, 32 dB, 30 dB, 28 dB, 25 dB 22 dB, oder sogar 20 dB nicht überschritten.

Dies kann unter anderem auch dadurch erreicht werden, dass das Gehäuse der Pumpe mit einem Schallschluckenden Material umhüllt oder ausgekleidet ist. Hierzu kommen beispielsweise Schäume wie Neopren oder ähnliches in Frage.

Das Gehäuse und/oder die innenliegende Technik kann aus einem nichtstarren Material angebracht bzw. aus einem solchen gefertigt ein.

Bevorzugt weist die Pumpe eine Förderrate zwischen 0,5 ml min⁻¹ und 100 ml min⁻¹ auf. Diese können beispielsweise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 oder 100 ml min⁻¹ betragen.

Bevorzugt sind Förderraten zwischen 2 ml min⁻¹ und 50 ml min⁻¹. Besonders bevorzugt sind Förderraten zwischen 10 ml min⁻¹ und 20 ml min⁻¹
Besagtes evakuiertes Gefäß kann ähnlich der bekannten Redonflasche an die erfindungsgemäße Vorrichtung zur Wundbehandlung angeschlossen werden und diese so unter Unterdruck setzen. Besagtes evakuiertes Gefäß weist eine flüssigkeitsabsorbierende Polymere enthaltende Einlage auf, bevorzugt in Form einer Wandauskleidung.

Besonders bevorzugt kann besagtes evakuiertes Gefäß in Form einer Patrone vorgesehen sein, die in eine Halterung eingelegt wird, die bereits mit der erfindungsgemäßen Drainagevorrichtung zur Wundbehandlung verbunden ist. Wenn die Patrone voll ist, wird sie entnommen und entsorgt, und eine neue evakuierte Patrone kann in die Halterung eingelegt werden.

Besonders vorteilhaft sind die vorgenannten Ausgestaltungen, weil durch Verzicht auf eine eigene Pumpe und stattdessen Verwendung eines evakuierten Gefäßes die Vorrichtung mobil und netzunabhängig wird, so dass der Patient selbst mobil wird. Darüber hinaus kann so eine kleinere Bauweise erzielt werden, die es dem Patienten ermöglicht, die Vorrichtung diskret zu verbergen. Vorteilhaft ist hierzu insbesondere eine anatomisch angepasste Ausgestaltung des besagten evakuierten Gefäßes bzw. der erwähnten Halterung, was ein unauffälliges Tragen derselben beispielsweise am Bein ermöglicht.

Ferner macht eine solche Vorrichtung keinerlei Betriebsgeräusche und ist sehr leicht zu bedienen.

Ähnliches gilt für die erwähnte Handbetriebene Unterdruckquelle. Hier kann es sich im einfachsten Falle um eine Plastikspritze mit ausreichend hohem Volumen handeln. Andere Möglichkeiten liegen in einer einem Gummiball ähnlichen Pumpe, einem Faltenbalg etc.

Das Wundabdeckungselement dient dazu, die Wunde so zu verschließen, dass ein Unterdruck erzeugt werden kann. Dabei kann optional vorgesehen sein, dass das Wundabdeckungselement elastisch ausgebildet ist (beispielsweise durch Verwendung eines Silikons oder eines elastischen Polyurethans). Auf diese Weise wird bei Anlegen eines Unterdrucks die Wundpflegevorrichtung an den Wundgrund gepresst und so ein Kontakt zwischen Wundgrund und Absorptionskörper oder Wundkontaktschicht hergestellt. Dies allein kann schon ausreichen, um eine aktive Akquirierung von Wundexsudaten aus der Tiefe der Wunde zu stimulieren. In diesem Fall wird die Hauptlast der Flüssigkeitsakquirierung durch den Superabsorber getragen. Darüber hinaus kann so gewährleistet sein, dass die volle Aufnahmekapazität des Absorptionskörpers ausgeschöpft werden kann, weil das Abdeckungselement der flüssigkeitsbedingten Volumenzunamhe des Absorptionskörpers nachgeben kann.

Besonders bevorzugt ist vorgesehen, dass die Einrichtung zur Erzeugung von atmosphärischem Unterdruck in einem Flachgehäuse (10) untergebracht ist.

Dabei ist bevorzugt vorgesehen, dass das Flachgehäuse (10) an seiner dem Wundabdeckungselement (1) zugewandten Flachseite (13.1) wenigstens teilweise mit einer Klebefläche (14.1) versehen ist, die wiederum mit einem abziehbaren Schutzfolienelement (15) abgedeckt ist, so dass nach Abziehen des Schutzfolienelementes (15) eine an der besagten Flachseite (13.1) des Flachgehäuses (10) befindliche Ansaugöffnung (16) mit Rückschlagventil freigegeben ist, die mit der Öffnung (3) am Wundabdeckungselement (1) koinzidiert.

Dabei kann ferner das Flachgehäuse (10) an seiner dem Wundabdeckungselement (1) zugewandten Flachseite (13.2) wenigstens teilweise mit einer Klebefläche (14.2) versehen ist, die wiederum mit einem abziehbaren Schutzfolienelement (15) abgedeckt ist, so dass nach Abziehen des Schutzfolienelementes (15) das Flachgehäuse (10) auf das Wundabdeckungselement (1) aufgeklebt werden kann.

Bevorzugt weist das Flachgehäuse eine maximale Dicke von 25 mm auf. Besonders bevorzugt beträgt die Dicke nicht mehr als 22 mm, 20 mm, 18 mm, 15 mm, 12 mm, 10 mm oder sogar 8 mm.

Bevorzugt ist weiterhin vorgesehen, dass Vorrichtung ferner eine wundzugewandte flüssigkeitsdurchlässige Wundkontaktschicht aufweist.

Wichtig ist hierbei, dass der Wundexsudate aufsaugende Absorptionskörper und die wundzugewandte flüssigkeitsdurchlässige Wundkontaktschicht auch luftdurchlässig sein müssen, sodass der angelegte Unterdruck sich auch in den Wundberiech fortsetzen kann. Hierfür können ggf. Stanzungen, Poren, Schlitze oder dergleichen vorgesehen sein.

Bevorzugt handelt es sich bei einer solchen Wundkontaktschicht um ein Kunststoffgitter (bevorzugt aus massivem Silikon oder aus einem silikonbeschichteten Material), einen Schaumstoff (bevorzugt gelocht, oder in Form eines als Granufoam bekannten offenporigen Schaumstoffs), ein Abstandsgewirke, ein Gitter aufweisend Silber- oder Kupferdrähte bzw. bestehend aus Polymerfasern oder Drähten, die Silber- oder Kupfer bzw. deren Ionen enthalten, eine Schicht enthaltend Silikon oder ein Hydrokolloid nebst Lochungen, eine Schicht enthaltend modifizierte Cellulose, bevorzugt um Derivate der Cellulose wie beispielsweise Carboxymethylcellulose oder deren Derivate, und/oder eine perforierte Folie.

Bevorzugt ist ein dreidimensionales Wunddistanzgitter vorgesehen, wie es beispielsweise unter dem Markennamen "sorbion plus" am Markt und in der EP2004116A1 beschrieben ist. Ein solches Wunddistanzgitter verhindert die Eingranulierung und ermöglicht damit einen atraumatischen Verbandwechsel, hat ferner eine Biofilmauflösende Wirkung und außerdem einen Ventileffekt, dergestalt, dass ein Rückfluss von Exsudat reduziert wird.

Die Anmelderin konnte zeigen, dass diese Eigenschaften, die aus "passiven" Wundauflagen bekannt sind, auch bei den hier beschriebenen "aktiven" vakuumunterstützten Wundauflagen hervorragende Vorteile bieten.

In ähnlicher Weise können die für die besagte Wundkontaktschicht verwendbaren Materialien auch auf der wundabgewandten Seite, beispielswiese unmittelbar unterhalb des Wundabdeckungselements, zum Einsatz kommen, um beispielsweise den Unterdruck ebenmäßig zu verteilen.

Besonders bevorzugt ist vorgesehen, dass zwischen der Einrichtung zur Erzeugung von atmosphärischem Unterdruck und dem Wundsekrete aufsaugenden Absorptionskörper eine Barriere angeordnet ist, die keine Flüssigkeiten passieren lässt. Auf diese Weise wird sichergestellt, dass keine Flüssigkeit in die Pumpe gelangt. Letztere verbleibt innerhalb der Wundabdeckung und wird von Wundexsudate aufsaugenden Absorptionskörper aufgenommen
Besagte Barriere weist bevorzugt eine semipermeable Membrane auf, beispielsweise aus einem Material wie Goretex, etc. Es kann sich ebenso um ein hydrophobes Filterlement handeln, das beispielsweise eine Wassersäule von 120 cm oder höher aufweist. Ferner kann ein Partikelfilter vorgesehen sein, der beispielsweise eine Porengröße kleiner 1 µm, bevorzugt kleiner 0,2 µm aufweist. Auf diese Weise kann die Evakuation von Infektiösen Partikeln verhindert werden. Im Idealfall ist besagter Partikelfilter in der Flüssigkeitsbarriere verwirklicht.

Besonders bevorzugt ist ferner vorgesehen, dass die Einrichtung zur Erzeugung von atmosphärischem Unterdruck auf der wundabgewandten Seite des Wundabdeckungselements angeordnet ist.

Diese Ausführungsform bietet als Vorteile ein leichteres Austauschen der Pumpe bzw. der Stromversorgung, Steuer- oder Spannungsversorgungsleitungen müssen nicht durch das Wundabdeckungselement hindurchgeführt werden, Einrichtung zur Erzeugung von atmosphärischem Unterdruck wird weniger leicht kontaminiert, was eine Wiederverwendung erleichtert.

Alternativ ist vorgesehen, dass die Einrichtung zur Erzeugung von atmosphärischem Unterdruck auf der wundzugewandten Seite des Wundabdeckungselements angeordnet ist. Auf diese Weise bietet sich ein einheitlicheres Erscheinungsbild, und der Patient kann beispielsweise Duschen, während er die Vorrichtung am Körper trägt.

Bevorzugt ist vorgesehen, dass die Einrichtung zur Erzeugung von atmosphärischem Unterdruck mindestens eine Pumpe aufweist. Besonders bevorzugt kann hierbei vorgesehen sein, dass die Einrichtung zur Erzeugung von atmosphärischem Unterdruck zwei oder mehr parallel oder in Reihe angeordnete Pumpen aufweist.

Bei Parallelanordnung kann so die Fördermenge erhöht - im Idealfall verdoppelt - werden, während bei Reihenanordnung der maximale erzeugbare Unterdruck erhöht - im Idealfall verdoppelt - werden kann.

Auf diese Weise können beispielsweise zwei Pumpen, die jeweils in der Lage sind, einen maximalen Unterduck von -80 mm HG zu erzeugen, in Reihe angeordnet einen maximalen Unterdruck von -160 mm Hg erzeugen. Dies ist insbesondere für die Verwendung von miniaturisierten Pumpen, die im folgenden noch beschrieben werden, vorteilhaft, da diese häufig in Soloanordnung nur einen relativ geringen Unterdruck erzeugen können, der ggf. für die klinische Anwendung nicht ausreichend ist. Vorteilhaft ist dabei ferner, dass der Stromverbrauch der Vorrichtung hierdurch nicht verdoppelt wird, da in der Vorrichtung neben der Pumpe noch andere Stromverbraucher existieren, z.B. die Steuerelektronik oder das Interface. Daher führt eine Verdoppelung der Leistung durch Parallel- oder Reihenanordnung zweier Pumpen nicht zu einer Verdoppelung des Stromverbrauchs.
***

In einer besonders bevorzugten Ausgestaltung ist vorgesehen, dass die Einrichtung zur Erzeugung von atmosphärischem Unterdruck wenigstens ein Steuermodul (12) umfasst. Insbesondere kann hier ein Drucksensor vorgesehen sein, der die dafür sorgt, dass bei einer Undichtigkeit oder Leckage frühzeitig durch Nachpumpen ein ausreichend hoher Unterdruck wieder hergestellt wird.

Das Steuermodul kann auch so ausgebildet sein, dass es vom Rest der Vorrichtung ablösbar ausgebildet ist, und beispielsweise als Fernbedienung fungiert.

Weiterhin ist bevorzugt vorgesehen, dass die Vorrichtung ferner mindestens eine Batterie umfasst. Besagte Batterie ist bevorzugt ausgewählt aus der Gruppe enthaltend
- Herkömmliche Einwegbatterien
- Herkömmliche wiederaufladbare Batterien
- Flat-Pack-Batterien
- Arrays von Kleinbatterien, bevorzugt "Knopfzellen"
- Flexible, biegsame wiederaufladbare Akkumulatoren

Besonders bevorzugt werden in einer Ausführungsform mit herkömmlichen Einwegbatterien bzw. herkömmlichen wiederaufladbaren Batterien der Typen Lady, AAA oder AAAA oder CR 1/3 N verwendet.

Die besagten Arrays von Kleinbatterien und die flexiblen, biegsamen wiederaufladbare Ackumulatoren, die beispielsweise auf Basis von Polymeren oder Papier, die größtmögliche mechanische Flexibilität bei nur wenigen Millimetern Dicke gewährleisten, oder als extrem dünne Lithium-Ionen-Akkus oder Metallhydrid-Akkus ausgestaltet sind, bieten den Vorteil, dass sie ausreichend hohe Batteriekapazität bei gleichzeitig hoher Flexibilität und geringer Dicke bieten, was den Benutzerkomfort erheblich erhöht.
Die Akkus können in Form eines Steckmoduls in das Gehäuse der Pumpe integriert sein.

Besonders bevorzugt ist vorgesehen, dass die Einrichtung zur Erzeugung von atmosphärischem Unterdruck unmittelbar am Wundabdeckungselement angeordnet ist. Auf diese Weise ist ein gesonderter Vakuumschlauch, der fertigungstechnische Probleme (ausreichend hohe Steifheit, um nicht unter Vakuum zu kollabieren) sowie hygienische Probleme (Gefahr der Kontamination) aufweist, verzichtbar, bzw. kann sehr kurz gehalten werden.

Ferner ist bevorzugt vorgesehen, dass die Vorrichtung eine Anschlussstelle für eine externe Stromversorgung aufweist. Eine solche Externe Stromversorgung kann einerseits dazu dienen, die wiederaufladbaren Batterien bzw. Akkumulatoren der Vorrichtung wieder aufzuladen. Ferner kann aber auch vorgesehen sein, dass die Vorrichtung direkt mit Strom aus dieser Stromversorgung betrieben werden kann.

Dies ist insbesondere dann vorteilhaft, wenn unmittelbar nach Anbringung der Vorrichtung das initiale Vakuum mit Strom aus der externen Stromversorgung eingestellt wird. Für die - weniger energieintensive - Aufrechterhaltung des Vakuums während des Betriebs der Vorrichtung kann dann Batterie- oder Akkustrom verwendet werden.

Bevorzugt kommt dabei beispielsweise eine externe Stromversorgung über eine Micro-USB-Anschlussbuchse und ein entsprechendes Netzgerät in Betracht. In dieser Ausführungsform kann außerdem die Anschlussbuchse als Schnittstelle für einen PC dienen, um Daten auszutauschen, beispielsweise betreffend den Betriebszustand der Pumpe, ein Betriebsprotokoll der Pumpe, Unterdruckwerte oder aber auch physiologische Werte (siehe unten)

Alternativ kann die externe Stromversorgung auch über ein über 220 V Steckernetzteil oder über eine 12V-Stromversorgung erfolgen.

Im wietesten Sinne kann es sich bei der Anschlussstelle für eine externe Stromversorgung auch um eine Verbindung zu einem Solarpanel sein, mit dessen Hilfe die Einrichtung zur Erzeugung von Unterdruck betrieben bzw. eine etwaig vorhandene aufladbare Batterie wieder aufgeladen wird.

Weiterhin ist bevorzugt vorgesehen, dass zwischen der Einrichtung zur Erzeugung von atmosphärischem Unterdruck und dem Wundabdeckungselement bzw. dem Wundexsudate aufsaugenden Absorptionskörper eine Kupplung, ein Absperrventil und/oder ein Dreiwegehahn angeordnet ist.

Mittels dieser Vorrichtung kann gewährleistet werden, dass a) die Einrichtung zur Erzeugung von atmosphärischem Unterdruck vom Rest der Vorrichtung abgekoppelt werden kann, b) ein einmal gezogener Unterdruck möglichst lange gehalten wird und/oder c) zwecks Schonung der Batterie oder bei entladender Batterie über eine Externe Pumpe oder ein externes Vakuumgefäß initial oder erneut ein Unterdruck angelegt werden kann.

Besagte externe Pumpe bzw. besagtes externes Vakuumgefäß kann beispielsweise in der Klinik oder beim Patienten zu Hause stehen, oder aber in mobiler Form ausgebildet sein (beispielsweise als in Form eines Koffers, integriert in die Kleidung des Patienten oder über einen Gurt am Patienten befestigt). So wird sichergestellt, dass die Vorrichtung an der Wunde klein und unauffällig bleibt - da ihre Aufgabe vor allem die ist, das Vakuum aufrecht zu erhalten, bei Bedarf jedoch ausreichend Pumpkapazität zur Verfügung steht, um eine effiziente Therapie zu gewährleisten.

Weiterhin ist bevorzugt vorgesehen, dass die Vorrichtung ferner einen Sensor für mindestens einen physiologischen und/oder pathologischen Parameter des Wundexsudats aufweist. Bevorzugte physiologische und /oder pathologische Parameter sind
a) pH-Wert des Exsudats
b) Proteingehalt
c) Keimbelastung
d) Anteil an Blut bzw. Blutzellen in der abgeführten Flüssigkeit, und/oder
e) Sauerstoffgehalt.

Hier können beispielsweise ein pH-Meter, eine Sauerstoffsonde, ein Keimdetektor, ein Hämoglobinsensor oder ein Proteinsensor zu Einsatz kommen.

Ein Arzt kann dann beispielsweise sofort erforderliche Maßnahmen ergreifen, beispielsweise eine Antibiose oder die topische Gabe von antimikrobiellen Agenzien (beispielsweise Silber, Kupfer, Octenidin, Antibiotika, etc) oder Puffern einleiten oder eine geeignete Kochsalz-, Ringer oder Eiweißlösung verabreichen. Bei Anwesenheit von Blut bzw. Blutzellen in der abgeführten Flüssigkeit kann ein Alarm ausgelöst und/oder der Pumpvorgang unterbrochen werden, um eine akute Anämie zu verhindern.

Auch kann vorgesehen sein, dass die Vorrichtung Empfehlungen zur Wundspülung ausspricht, und diese dann - ggf nach Bestätigung durch den Arzt - selbständig durchführt. Hierzu kann über Pumpumkehr Spülmedium aus einem Behälter in die Wunde gebracht werden. Besagte Spüllösung kann insbesondere die oben genannten Agenzien oder Lösungen enthalten.

Besonders bevorzugt ist vorgesehen, dass erfindungsgemäße Vorrichtung eine Einrichtung zur Bestimmung der Keimbelastung und/oder der stofflichen Zusammensetzung des Exsudats aufweist. Eine solche Vorrichtung kann beispielsweise ein Biosensor (z.B. ein Biochip) oder ein Teststreifen mit Farbindikatoren sein.

In Bezug auf die Keimbelastung sind hier insbesondere multiresistente Keime von Interesse, die u.a. für die berüchtigten Krankenhausinfektionen verantwortlich sind, beispielsweise MRSA (Methicillin-resistenter Staphylococcus aureus, ORSA (Oxacillin-resistenter Staphylococcus aureus), VISA (Vancomycin-intermediate Staphylococcus aureus) oder VRSA (Vancomycin-resistenter Staphylococcus aureus). Solche Biosensoren beruhen beispielsweise auf dem Prinzip des Immunoassays oder dem eines Biochips - bevorzugt in Form eines Einweg-Produkts, oder auf Aptameren. Dem Fachmann sind solche Technologien aus der einschlägigen Literatur bekannt.

In Bezug auf die stoffliche Zusammensetzung des Exsudats sind insbesondere Serumproteine (Albumine), Matrix-Metalloproteasen, Gerinnungsfaktoren und -Proteine (beispielsweise Thrombin, Fibrin), Entzündungsmarker (Cytokine), Insulin, oder Glucose von Interesse, ebenso die für Nieren- und Leberwerte typischen Marker. Auch hier kann beispielswiese das Immunoassay-Prinzip verwirklich sein, bevorzugt in Form eines Einweg-Produkts (Teststreifen).

Bevorzugt weist besagter Biochip, bevorzugt in Kombination mit dem weiter unten beschriebenen Bedienungs- und/oder Steuerelement, einen Speicher auf, in den die Messwerte zur Keimbelastung und/oder stofflichen Zusammensetzung des Exsudats eingelesen und aus welchem diese auch wieder ausgelesen werden können. Auf diese Weise kann der zeitliche Verlauf der Zusammensetzung des Exsudats verfolgt und protokolliert werden.

Besonders bevorzugt ist ferner vorgesehen, dass der Absorptionskörper (20) mit einer flüssigkeitspermeablen Hülle (22) umgeben ist.

Ebenso kann vorgesehen sein, dass die Vorrichtung ferner einen Abstandskörper (23) aufweist. Dieser kann bevorzugt zwischen Wunde und Wundabdeckungselement platziert werden, ebenso aber auch - wenn vorhanden - zwischen Absorptionskörper und Wundabdeckungselement bzw. zwischen Wunde und Absorptionskörper platziert werden.

Besonders bevorzugt ist vorgesehen, dass das Wundabdeckungselement (1) flüssigkeits- und/oder gasundurchlässig ist. Auf diese Weise wird das Anlegen eines Vakuums ermöglicht, und es wird verhindert, dass Flüssigkeit, die u.U, kontaminiert sein kann, austritt.

Bevorzugt ist besagtes Wundabdeckungselement elastisch ausgebildet.

Besagtes Wundabdeckungselement weist überdies bevorzugt mindestens eine Klebeeinrichtung auf, mittels derer die Vorrichtung an der die Wunde umgebenden haut eines Patienten angebracht werden kann. Besagte Klebeeinrichtung sorgt überdies bevorzugt auch für eine flüssigkeits- und/oder gasundurchlässige Verbindung des Wundabdeckungselements mit der Haut. Auf diese Weise wird sichergestellt, dass ein Unterdruck dauerhaft angelegt werden kann.

Als Klebstoffe kommen hier insbesondere Silikonkleber, Hydrokolloidkleber und dergleichen in Frage.

Besonders bevorzugt ist ferner vorgesehen, dass das Wundabdeckungselement wasserdampfdurchlässig ist. Bevorzugt ist das Wundabdeckungselement ferner transparent ausgebildet. Ferner ist bevorzugt, dass das Wundabdeckungselement ein Fenster zur Entnahme des Absorptionskörpers aufweist. Dies ist sinnvoll, falls die Wunde stark exsudiert, da dann der Absorptionskörper ausgewechselt werden, die Vorrichtung aber am Patienten verbleiben kann.

Bevorzugt ist ferner vorgesehen, dass das Wundabdeckungselement (1) eine Einrichtung zur Befestigung der Einrichtung zur Erzeugung von Unterdruck aufweist. Dies kann beispielsweise eine ggf. der Flächengröße der Einrichtung zur Erzeugung von atmosphärischem Unterdruck bzw. ihres Gehäuses entsprechende Klebefläche (26) sein. Alternativ kann ein Druckknopf- oder ein Kletttverschlusssystem vorgesehen sein.

Ferner ist vorgesehen, dass der Absorptionskörper (20) mindestens eine superabsorbierende Substanz (24), wie eine modifizierte Cellulose und/oder ein Alginat aufweist.

Superabsorbierende Polymere (SAP) sind Kunststoffe, die in der Lage sind, ein Vielfaches ihres Eigengewichts - bis zum 1000-fachen - an Flüssigkeiten aufzusaugen. Chemisch handelt es sich dabei um ein Copolymer aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt"). Durch diese Brücken wird das Polymer wasserunlöslich. Beim Eindringen von Wasser oder wässrigen Salzlösungen in den Polymerpartikel quillt er auf und strafft auf molekularer Ebene dieses Netzwerk, so dass das Wasser ohne Hilfe nicht mehr entweichen kann. Die superabsorbierenden Polymere können in dem erfindungsgemäßen Wundpflegeartikel in Form eines Granulats, eines Pulvers, einer Schüttung, eines Presslings, eines Schaums, in Form von Fasern, eines Fasergewirkes, -geleges oder -vlieses und/oder einer Faserwatte vorliegen.

Alternativ können die Superabsorber auf Methylacrylsäurebasis, Polyvinylalkohol-Maleinsäureanhydrid-Copolymere, Polysaccharid-Maleinsäureanhydrid-Copolymere, Maleinsäurederivate, Acrylamidopropansulfonsäure-Copolymere, Stärke-Acrylnitril-Pfropfpolymere, gelantinisierte Stärkederivate, Alkyl- oder Hydroxyalkylcellulose, Carboxymethylcellulose, Stärke-Acrylsäure-Pfropfpolymere, Vinylacetat-Acrylsäureester- Copolymere, Acrylnitril- oder Acrylamid-Copolymere gewählt sein.

Im vorliegenden Kontext sind insbesondere die in der Literatur beschriebenen Matrix-Metalloproteasen modulierenden sowie keimhemmenden Eigenschaften superabrobierender Polymere von Interesse, da sie synergistisch die Wirkung der Unterdrucktheraüpie unterstützen.

So konnte gezeigt werden, dass ein Absorptionskörper der Firma Sorbion, der Superabsorbierende Polyacrylate enthält, in seiner keimhemmenden Wirkung einem Silber enthaltenden Absorptionskörper, wie er in ähnlicher Form in anderen Unterdruck-Therapievorrichtungen verwendet wird, gleichkommt, freilich ohne die für Silber bekannten Nebeneffekte.

Hierfür ist allerdings ein Mindestanteil an superabsorbierenden Polymeren, wie andernorts hierin beschrieben, erforderlich.

Bei modifizierter Cellulose handelt es sich bevorzugt um Derivate der Cellulose, bevorzugt Sulfoalkylierte Cellulose und deren Derivate, bevorzugt Celluloseethylsulfonate (sogenannte "Durafaser"), Carboxyalkylierte Cellulose, bevorzugt Carboxymethylzellulose (sogenannte "Hydrofaser"), Carboxyethylcellulose und/oder Carboxypropylcellulose, Komplexere Cellulosederivative, wie Sulphoethylcarboxymethylcellulose, Carboxymethylhydroxyethylcellulose, Hydroxy-propyl-methylcellulose, und Amidierte Cellulosederivate, wie Carboxymethylcellulose-Amid oder Carboxypropylcellulose-Amid. Carboxymethylcellulose liegt insbesonderte in Form von Natriumcarboxymethylcellulose vor und ist unter dem Namen "Hydofaser" im Handel. In Hygiene- und Wundprodukten werden die Fasern in eine flächeige Matrix überführt. Durch die Aufnahme von Flüssigkeit aus dem Wundexsudat werden die Fasern nach und nach in ein Gelkissen umgewandelt, das die Flüssigkeit hält und nicht wieder freigibt. Dabei sind die Fasern so aufgebaut, dass das Wundexsudat nur in vertikaler Richtung aufgenommen wird. Dies bedeutet dass, solange die Kapazität reicht, das Exsudat nicht über den Wundrand fließt. Auf diese Weise kann eine Wundrandmazeration effektiv verhindert werden.

Bei besagten hydroaktiven Polymeren kann es sich auch um Alginate handeln. Alginate werden aus den Braunalgen gewonnen und zu einem faserigen Vlies verwoben Chemisch handelt es sich um Polysaccharide, und zwar Calcium- und/oder Natrimsalze der Alginsäuren. Alginate können bis zum 20fachen ihres Eigengewichtes an Flüssigkeit aufnehmen, dabei wird das Wundexsudat in die Hohlräume eingelagert. Die im Alginatgitter enthaltenen Ca2+ Ionen werden gegen die Na+ Ionen aus dem Exsudat ausgetauscht, bis der Sättigungsgrad an Na-Ionen im Alginat erreicht ist. Dabei kommt es zu einem Aufquellen der Wundauflage und zur Umwandlung der Alginatfaser in einen Gelkörper durch Aufquellen der Fasern.

Ebenso kann es sich bei besagten hydroaktiven Polymeren auch um Hydrogel-Nanopartikel aufweisend Hydroxy-Terminierte Methacrylatmonomere, wie 2-Hydroxyethylmethacrylat (HEMA) und/oder 2-Hydroxypropylmethacrylat (HPMA), die z.B. als Altrazeal vermarktet werden, handeln.

In einer weiteren bevorzugten Ausgestaltung ist vorgesehen, dass der Absorptionskörper einen Anteil von ≥ 40 Gew.-% an superabsorbierenden Polymeren aufweist. Besonders bevorzugt beträgt der Gewichtsanteil der superabsorbierenden Polymere ≥ 45, 50, 55, 60, 65 oder 70 Gew.-%.

Erfindungsgemäß ist vorgesehen, dass der Absorptionskörper ein Vlies aufweisend Zellulosefasern aufweist.

Der Absorptionskörper weist einen im Wesentlichen flachen Absorptionskörper aus Absorptionsmaterial auf, der aus einem aufsaugenden Vlies mit darin verteilten superabsorbierenden Polymeren besteht. Diese können in Form eines Granulats, eines Pulvers, einer Schüttung, eines Presslings, eines Schaums, in Form von Fasern, eines Fasergewirkes, - geleges oder -vlieses und/oder einer Faserwatte vorliegen.

Dabei weist der Absorptionskörper mindestens ein Material auf, das ausgewählt ist aus der Gruppe enthaltend eine Matte, insbesondere aus einem Airlaid aus besagten Garnen oder Fasern aus superabsorbierenden Polymeren mit eingearbeiteten superabsorbierenden Polymeren, und/oder eine lose Füllung aus superabsorbierenden Polymeren. Besagte Airlaidmatte kann bevorzugt einen im Wesentlichen flachen Materialabschnitt aus Absorptionsmaterial aufweisen, der z. B. aus einem aufsaugenden Vlies aus den genannten Fasern mit darin verteilten superabsorbierenden Polymeren besteht.

Dieser Absorptionskörper kann der absorbierenden Einlage entsprechen, die in einer Wundauflage der Anmelderin der vorliegenden Erfindung enthalten ist, wie sie beispielsweise in der WO03094813, der WO2007051599 und der WO0152780 offenbart ist und unter dem Handelsnamen "sorbion sachet" vertrieben wird. Der Offenbarungsgehalt der genannten Schriften sei dem Offenbarungsgehalt dieser Schrift vollumfänglich beigefügt.
Der Absorptionskörper kann in einer anderen Ausgestaltung ebenso einen Kern bilden, der - ggf. flockenartige - Fasern oder Garne aus superabsorbierenden Polymeren sowie superabsorbierenden Polymeren in Granulatform aufweist, wobei die Granulate an die Fasern bzw. Garne in mehreren Höhen angeklebt bzw. angeschweißt sind, und die Granulate über mehr als 50 % der gesamten Bauhöhe wenigstens eines Abschnitts des Kerns verteilt sind, wobei vermengte Bereiche von Granulat und Fasern vorliegen. Der Gewichtsanteil der superabsorbierenden Polymeren kann dabei bevorzugt im Bereich zwischen 10 - 25 Gew.-% liegen. Ähnliche Konstruktionen sind aus herkömmlichen Inkontinenzmaterialien bekannt und wie Hygienebinden für ihre polsternden Eigenschaften bekannt. Um besagten Kern herum kann eine

Hülle angeordnet sein, die in Bereichen überlappend angeordnet ist, und der z.B. eine Klebenaht überdeckt bzw. Teil derselben ist.

Besonders bevorzugt weist der Absorptionskörper ein Vlies, bevorzugt ein Nonwoven oder Airlaid, auf, dass aus Superabsorbierenden Fasern ("SAF", bevorzugt Polyacrylate) besteht oder diese als Bestandteil enthält. Die Fasern können beispielsweise mit Fluff Pulp (Zellulose) oder mit Polyesterfasern gemischt sein. Alternativ oder Zusätzlich kann ein Schichtaufbau vorgesehen sein.

Das Flächengewicht kann dabei im Bereich zwischen ≥ 50 und ≤ 2000 g/m² liegen. Bevorzugt sind dabei Flächengewichte von 50, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, und/oder 2000 jeweils +/- 25 g/m² vorgesehen.

Die Dicke kann dabei im Bereich zwischen ≥ 2 und ≤ 50 mm liegen. Bevorzugt sind dabei Dicken von 2, 4, 6, 8, 10, 12, 14, 16, 18, 20, 22, 24, 26, 28, 30, 32, 34, 36, 38, 40, 42, 44, 46, 48, und/oder 50 jeweils +/- 1 mm vorgesehen.
Die Aufnahmekapazität kann dabei im Bereich zwischen ≥ 3 und ≤ 30 ml 0.9 % Kochsalz/m² bei 0,2 psi Druck liegen. Bevorzugt sind dabei Werte von 3, 4, 5, 6, 7, 8, 9, 10, 11, 12, 13, 14, 15, 16, 17, 18, 19, 20, 21, 22, 23, 24, 25, 26, 27, 28, 29, und/oder 30 ml 0.9 % Kochsalz/m² vorgesehen. Alternativ kann die Aufnahmekapazität dabei im Bereich zwischen ≥ 2 und ≤ 50 g Wasser/g liegen. Bevorzugt sind dabei Werte von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, und/oder 50 g Wasser/g

Der Gesamtgehalt an Superabsorbierenden Polymeren kann dabei im Bereich zwischen ≥ 5 und ≤ 100 % w/w liegen. Bevorzugt sind dabei Werte von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 und/oder 100 % w/w.

Die Zugfestigkeit kann dabei im Bereich zwischen ≥ 5 und ≤ 80 N/5 cm liegen. Bevorzugt sind dabei Werte von 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 und/oder 80 N/5 cm.

Die Dehnbarkeit kann dabei im Bereich zwischen ≥ 10 und ≤ 80 % liegen. Bevorzugt sind dabei Werte von 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 und/oder 80 %.

In der Praxis haben sich folgende Typen als besonders vorteilhaft herausgestellt:

| **Typ** | **1** | **2** | **3** | **4** | **5** | **6** |
|---|---|---|---|---|---|---|
| Aufbau 1 | Schichtaufbau: Thermogeb ondetes Airlaid mit laminiertem Nonwoven | 40% Polyester Kurzschnittfaser; 60 % SAF | Bicompone ntenfaser aus SAF und einem Thermoplasten | Schichtaufbau: Thermogebondetes Airlaid mit laminiertem Nonwoven | 25 % Polyester; 75 % SAF | 40 % Polyester Kurzschnittfaser; 60 % SAF |
| Aufbau 2 | Bicompone ntenfaser aus SAF und einem Thermoplasten + Fluff-Pilp | Nadelfilz | Kardiertes, thermogebo ndetes Nonwoven | Bicomponentenfas er aus SAF und einem Thermoplasten + Fluff-Pilp | Nadelfilz | Nadelfilz |
| Typ der SAF Faser | 101/6/10 | 102/52/10 | 102/52/10 | 101/6/10 | | |
| Gewicht (g/m²) | 560 | 540 | 1000 | 350 | 150 | 380 |
| Dicke (mm) | 6 | 5,4 | 20 | 3,5 | 2,4 | 3,8 |
| Aufnahmekapazität | 31,21 Wasser/m² | > 20 g Wasser/g | > 16 g Wasser/g bzw. 16000 g Wasser/m² | 19,5 1 Wasser/m² | >25g 0.9 % Kochsalz/g | > 17 g Wasser/g bzw. 6400 g/m² |
| Aufnahmekapazität unter Druck (ml 0.9 % Kochsalz/m² bei 0,2 psi Druck) | 16 | | | 16 | | |
| Gesamtgehalt an Superabsorbierenden Polymeren (% w/w) | 18 | 40 | 50 | 18 | 75 | 60 |
| Zugfestigkeit (N/5cm) | | | | | 16±13 | 16±13 |
| Dehnbarkeit (%) | | | | | 60±18 | 60±18 |

Der Absorptionskörper kann in einer anderen Ausgestaltung ebenso mindestens eine flache Lagen aufweisend Fasern oder Garne aus superabsorbierenden Polymeren enthalten, an welche superabsorbierende Polymere in Granulatform geklebt sind. Dadurch ergibt sich in einer bevorzugten Ausgestaltung ein Aufbau des Körpers, der wenigstens drei Schichten aufweist, wobei zwei Deckschichten eine Schicht aufweisend superabsorbierende Polymere umgeben.

Dabei liegen in der Ebene keine Vermengungen von Fasern und superabsorbierenden Polymeren vor; sondern lediglich fixierte Benachbarungen beider Materialien. Die ggf. vorgesehenen mehreren Lagen können dabei in einer bevorzugten Ausgestaltung auch durch Walzen, Pressen, Kalandrieren oder ähnliche Verfahren physisch miteinander verdichtet sein.

Es gelten ähnlich bevorzugte Parameterbereiche wie oben genannt. Die Flüssigkeitsretention kann zwischen ≥ 5 und ≤ 100 g/g betragen. Bevorzugt sind dabei Werte von 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 und/oder 100 g/g

In der Praxis haben sich folgende Typen als besonders vorteilhaft herausgestellt:

| **Typ** | **1** | **2** | **3** | **4** | **5** | **6** | **7** | **8** |
|---|---|---|---|---|---|---|---|---|
| Gewicht (g/m²) | 450 | 300 | 150 | 50 | 100 | 120 | 140 | 440 |
| Dicke (mm) | 1,3 | 1,2 | 0,9 | 0,7 | 0,7 | 0,76 | 1 | 1,2 |
| Flüssigkeitsretention (g/g) | 28 | 33 | 28 | 15 | 25 | 28 | 11,5 | 38 |
| Zugfestigkeit (N/5cm) | 25 | 55 | 20 | 20 | 20 | 20 | 15 | 20 |
| Aufnahmekapazität (g/g) | 45 | 20 | 50 | 20 | 40 | 50 | 28 | 55 |

Überdies kann der Körper sich wiederholende Musterungen oder Maserungen aufweisen, wie z.B. ein Karomuster, ein Stanzmuster oder dergleichen.

Besonders bevorzugt weist besagter Absorptionskörper ein Flächenmass von 5 x 10, 5 x 20, 10 x 10, 10 x 15 oder 15 x 15 cm auf.

Besonders bevorzug ist ferner vorgesehen, dass besagter Absorptionskörper neben einer Lage aufweisend superabsorbierende Polymere mindestens eine zweite flankierende Lage aufweist, die weniger oder keine superabsorbierenden Polymere aufweist und flächenmäßig über erstgenannte hinausragt. Auf diese Weise wird sichergestellt, dass die Lage aufweisend superabsorbierende Polymere entsprechend der Flüssigkeitsaufnahme an Volumen gewinnen kann, ohne dass die Volumenzunahme nach außen hin erkennbar ist, weil letztere durch die zweite Schicht kaschiert wird.

Bevorzugt ist ferner vorgesehen, dass die Einrichtung zur Erzeugung von atmosphärischem Unterdruck in ihrer Pumprichtung reversibel ist. Auf diese Weise lässt sich die Einrichtung auch als steuerbare Dosierpumpe für Medikamente, Spüllösungen etc. einsetzen.

Ferner kann die Einrichtung zur Erzeugung von atmosphärischem Unterdruck ein Display oder Betriebszustandsanzeigen aufweisen, beispielsweise in Form eines LCD-, LED- oder TFT-Displays oder in Form von LED-Leuchten.

Diese können beispielsweise Betriebszustands-. Batteriezustands- oder Fehlermeldungen anzeigen oder andere Parameter darstellen.

Ferner ist bevorzugt vorgesehen, dass sich die Einrichtung zur Erzeugung von atmosphärischem Unterdruck bzw. deren Steuermodul über mindestens eine Einrichtung ausgewählt aus der Gruppe enthaltend
- Touchscreen
- Folientastatur
- Reedkontakt
- Fernbedienung
betätigen läßt

Das Touchscreen, die Folientastatur wie auch der Reedkontakt haben dabei den Vorteil der besseren Hygiene, da erstere beide leichter zu hygienisieren ist als eine herkömmliche Tastatur und letzteres ohne zugängliche Kontakte oder Leitungen auskommt. Besagte Fernbedienung ist beispielsweise nach dem IR-Standard, oder dem Bluetooth-Standard aufgebaut oder entspricht in ihrer Funktionsweise einer Funkfernbedienung.

Das Touchscreen kann dabei so verwirklicht sein, dass es nahtlos in das Gehäuse integriert ist und erst bei Aktivierung für den Benutzer sichtbar wird.

Das Touchscreen oder das Display kann dabei auch flexibel oder biegsam ausgestaltet sein. Besonders bevorzugt kann überdies vorgesehen sein, dass die Einrichtung zur Erzeugung von atmosphärischem Unterdruck bzw. deren Steuermodul eine klapp - oder slidefähige Bedienungseinheit bzw. einen entsprechende Akku bzw. eine entsprechende Batterie aufweist, ähnlich wie bei entsprechenden Mobiltelefonen. Dies hat neben ästhetischen auch hygienische Vorteile. Ferner können so zwei Bedienungslevels verwirklicht werden, nämlich ein für den Patienten gedachtes Bedienungslevel, das die grundlegenden Funktionen zugänglich macht, und ein für das Pflegepersonal oder den Servicetechniker gedachtes Bedienungslevel, dass weitere Betriebs- und Wartungsfunktionen erschließt.

Ferner ist bevorzugt vorgesehen, dass das Wundabdeckungselement (1) eine Markierung (25) aufweist, die der Flächengröße der Einrichtung zur Erzeugung von atmosphärischem Unterdruck bzw. seines Gehäuses entspricht.

Weiterhin ist bevorzugt vorgesehen, dass
a) die Einrichtung zur Erzeugung von atmosphärischem Unterdruck so ausgestaltet ist, dass sie in einem inversen Betriebsmodus auch Überdrücke erzeugen kann, und/oder
b) die Vorrichtung ferner eine Einrichtung zum Eindosieren von Medikamenten bzw. zur Einleitung von Spülmedium aufweist.

Erstere Ausführungsform kann Beispielsweise für eine zeitweise Überdrucktherapie, verwendet werden, beispielsweise zur Wundkompression und/oder zur Blutungsstillung. Letztere Ausführungsform ist bereits oben beschrieben.

Ferner ist ein Verfahren zum Betrieb einer Wundpflegevorrichtung offenbart, in dem:
a) mithilfe der Einrichtung zur Erzeugung von atmosphärischem Unterdruck zunächst ein gegebener Unterdruck erzeugt wird,
b) die Einrichtung zur Erzeugung von atmosphärischem Unterdruck dann abschaltet, und
c) erst bei über- oder Unterschreiten eines Schwellen-Unterdrucks die Einrichtung zur Erzeugung von atmosphärischem Unterdruck erneut anschaltet und eine gegebenen Unterdruck erzeugt.

Weiterhin ist vorgesehen die Verwendung einer Wundpflegevorrichtung gemäß der Erfindung zur Behandlung von Wunden aufweisend Weichteildefekte, infizierten Wunden nach chirurgischem Debridement ("sogenanntes Erhaltungsdebridement"), aktives Wunddebridement, Lymphfisteln, sternalen Wundinfektionen, Thoraxwandfenstern, Dekubitus, Ulcus cruris, chronischen Wundheilungsstörungen, Strahlenulkus, Abdominellem Kompartmentsyndrom, septischem Abdomen, enteraler Fisteln und/oder Wunden, die durch ein oder mehrere Ödeme hervorgerufen werden, und/oder zur Fixierung von Hauttransplantaten und/oder zur Wundkonditionierung.

Dabei ist insbesondere an die Behandlung entzündlicher Ödeme oder durch chromische Veneninsuiffizienz hervorgerufenen Ödeme gedacht.

Die durch die Unterdrucktherapie ausgeübten Effekte beruhen teils auf sogenannten "Macrostrain"- und "Microstrain"-Effekten (Makrodeformation und Mikrodeformation). Bei Unterdruck übt die erfindungsgemäße Wundpflegevorrichtung über ihre Wundkontaktschicht (beispielsweise ein Silikongitter, ein Schaum, ein Zellulosevlies oder ein dreidimensionales Wunddistanzgitter) mechanische und biologische Kräfte auf die Wunde aus. So wird ein Milieu erzeugt, das die Wundheilung begünstigt. Makrodeformation ist die sichtbare Veränderung, die auftritt, wenn der Unterdruck den Schaumstoff kontrahiert. Makrodeformation zieht die Wundränder zusammen, stellt direkten und umfassenden Kontakt zwischen Wundbett und Wundkontaktschicht her, verteilt den Unterdruck gleichmäßig und entfernt Exsudat und infektiöses Material (Debridement). Mikrodeformation findet auf Zellebene statt. Dabei werden die Zellen gedehnt. Mikrodeformation reduziert die Ödembildung, fördert die Durchblutung, erhöht die Zellproliferation und Migration und fördert die Bildung von Granulationsgewebe

Weiterhin ist vorstellbar die Verwendung einer erfindungsgemäßen Wundpflegevorrichtung gemäß zur postoperativen Wund- oder Nahtversorgung. Dieser auch als Inzisionsmanagment bezeichnete Ansatz reduziert das Risiko für postoperative Komplikationen. Dabei trägt die Therapie dazu bei, Wundränder zusammenzuhalten, was wiederum die Wahrscheinlichkeit von Nahtdehiszenzen verringert. Ferner werden so OP-bedingte Gewebespannung und Ödeme reduziert und die Wunde vor externer Kontamination geschützt.

Diese Vorteile kommen insbesodnere beim ambulanten Einsatz zum Tragen, etwa in der Unfallmedizin oder im militärischen Feldeinsatz.

Ferner ist die Verwendung einer Wundpflegevorrichtung gemäß einem der vorhergehenden Ansprüche in einem Kompressionsverband vorgesehen.

Als Kompressionsverband wird ein textiler, elastischer Wickelverband bezeichnet. Es werden verschiedene Kompressionstechniken (Pütter, Fischer, Kornährenverband) unterschieden. Die Kompression des Gewebes hat folgende Wirkungen:
- Einengung der suprafaszialen Venen mit Wiederherstellung der Klappenfunktion
- Steigerung des Gewebedruckes mit höherer Resorption der Gewebsflüssigkeit in die Lymphgefäße, und
- Verbesserung der Muskelpumpe (durch Verstärkung des Widerlagers für die Muskulatur)
- Indikationen sind beispoeislweise Ödeme, primäre Varikosis, Thrombophlebitis, postthrombotisches Syndrom, Ulcus cruris

Für das Anlegen eines Kompressionsverbandes werden Kurzzugbinden, Verbandsklammern oder Heftpflaster, eine Schere und ggf. Polstermaterial benötigt.

Nach hygienischer Händedesinfektion werden die benötigten Materialen vorbereitet und der Patient in Rückenlage gelagert. Zu Beginn müssen die Extremitäten hochgehalten oder ggf. herzwärts ausgestrichen werden um einen venösen Blutstau zu beseitigen. Knochenvorsprünge wie Schienbein, Knöchel und Mulden wie die Kniekehle können entsprechend gepolstert werden. Der Fuß sollte nun im Sprunggelenk in 90°-Stellung gebracht werden. Beim Umwickeln sollte die Ferse miteingewickelt werden, da es sonst zu einem Fensterödem kommen kann. Die Kompression soll von distal nach proximal gleichmäßig abnehmen. Beim Anwickeln soll die Binde bis zu 3/4 der maximalen Dehnungsfähigkeit gedehnt werden. Jede Binde soll die nächste etwa zur Hälfte überlappen. Bei Gelenken kann in Form einer 8 um das Gelenk gewickelt werden, um eine Faltenbildung zu vermeiden (Kornährenverband). Ein Beispiel für einen solchen Verband ist in Fig. X gezeigt.

Erfindungsgemäß kann nun vorgsehen sein, die Wundpflegevorrichtung mit darauf angebrachter Einrichtung zur Erzeugung von atmosphärischem Unterdruck in den Kompressionsverband einzuarbeiten. Auf diese Weise kann ein Synergismus zwischen Kompressions- und Unterdrucktherapie erzielt werden.

Durch die optionale Fernsteuerbarkeit der Einrichtung zur Erzeugung von atmosphärischem Unterdruck kann dabei eine stete Kontrolle der Unterdrucktherapie auch bei fehlender Zugänglichkeit der Einrichtung zur Erzeugung von atmosphärischem Unterdruck gewährleistet werden.

Weitere Ausführungsformen werden nachfolgend durch die Abbildungen und Versuche verdeutlicht, durch deren konkrete Ausgestaltung die Erfindung jedoch in keiner Weise eingeschränkt wird.

Die Figuren zeigen:
- Fig. 1: eine Wundpflegevorrichtung gemäß Erfindung, in einer schematischen Schnittdarstellung;
- Fig. 2: eine Wundpflegevorrichtung gemäß einer anderen Ausführungsform, aufweisend einen Schaumstoff-Absorptionskörper, ebenso in einer schematischen Schnittdarstellung;
- Fig. 3: eine Mikropumpe in einer perspektivischen Ansicht auf ihre Unterseite;
- Fig. 4: die Mikropumpe gemäß Fig. 3 in einer schematischen Draufsicht auf ihre Oberseite;
- Fig. 5: eine andere Ausführungsform der Mikropumpe in einer schematischen Draufsicht auf ihre Oberseite;
- Fig. 6: Schema einer Markierung am Wundabdeckungselement;
- Fig. 7: eine weitere Ausführungsform der Wundpflegevorrichtung, in einer schematischen Schnittdarstellung;
- Fig. 8: Schema eines Mini-USB-Anschlusses an die Mikropumpe,
- Fig. 9a: einen Flachadapter in einer Explosionsdarstellung;
- Fig. 9b: den über das Wundabdeckungselement zusammengedrückten Flachadapter;
- Fig. 9c: Montage der Mikropumpe am Flachadapter mit schematisch angedeutetem Absorptionskörper;
- Fig. 10a: eine magnetische Verbindung der Mikropumpe mit dem Flachadapter, in einer schematischen Darstellung;
- Fig. 10b: eine zweite Ausführungsform der magnetischen Verbindung, mit einer innerhalb des Absorptionskörpers untergebrachten Mikropumpe, in einer schematischen Schnittdarstellung;
- Fig. 11a: eine andere Ausführungsform der Wundpflegevorrichtung, mit aufgeteilten Feldern des Wundabdeckungselementes, in Draufsicht auf das Wundabdeckungselement;
- Fig. 11b: einen Schnitt A-A gemäß Fig. 11a;
- Fig. 12: eine weitere Ausführungsform der Wundpflegevorrichtung, ebenso mit aufgeteilter Fläche des Wundabdeckungselementes, in Draufsicht auf das Wundabdeckungselement;
- Fig. 13a: eine weitere Ausführungsform der Wundpflegevorrichtung, in Draufsicht auf das Wundabdeckungselement;
- Fig. 13b: die Wundpflegevorrichtung gemäß Fig. 13a in einer schematischen Seitenansicht;
- Fig. 14: ein Wundabdeckungselement aufweisend Flachbatterien, in Draufsicht;
- Fig. 15: eine andere Ausführungsform des Wundabdeckungselementes mit Flachbatterie, in einer schematischen Draufsicht;
- Fig. 16a: eine weitere Ausführungsform der Wundpflegevorrichtung, in Draufsicht auf ein rundes Wundabdeckungselement;
- Fig. 16b: die Wundpflegevorrichtung gemäß Fig. 16a in einem schematischen Schnittdarstellung;
- Fig. 17a: einen schaumartigen Absorptionskörper in einer perspektivischen Ansicht;
- Fig. 17b: den Absorptionskörper gemäß Fig. 18a in einer Schnittdarstellung;
- Fig. 17c: den Absorptionskörper gemäß Fig. 18b mit einmontierter Mikropumpe;
- Fig. 17d: eine Wundpflegevorrichtung aufweisend den schaumartigen Absorptionskörper und Mikropumpe, in einer Schnittdarstellung;
- Fig. 18: eine weitere Ausführungsform der Wundpflegevorrichtung, in einer Schnittdarstellung;
- Fig. 19a: eine weitere Ausführungsform der Wundpflegevorrichtung, aufweisend einen Drainagekollektor, in Draufsicht,
- Fig. 19b: einen Schnitt B-B gemäß Fig. 19a;
- Fig. 19c: die Wundpflegevorrichtung gemäß Fig. 19a in Draufsicht auf ihre wundzugewandte Seite;
- Fig. 20: eine weitere Ausführungsform der Wundpflegevorrichtung, aufweisend ein Drei-Wege-Ventil, in einer schematischen Schnittdarstellung;
- Fig. 21: eine weitere Ausführungsform der Wundpflegevorrichtung, aufweisend zwei Mikropumpen, in Draufsicht auf das Wundabdeckungselement.
- Fig. 22: eine weitere Ausführungsform der Wundpflegevorrichtung, aufweisend einen zwischen der Mikropumpe und dem Wundabdeckungselement angeordneten Saugkopf, in einer schematischen Schnittdarstellung;
- Fig. 23: den in Fig. 17a gezeigten schaumartigen Absorptionskörper, mit mehreren Saugköpfen, in einer schematischen Schnittdarstellung;
- Fig. 24: eine Wundpflegevorrichtung mit eingebautem Absorptionskörper gemäß Fig. 23;

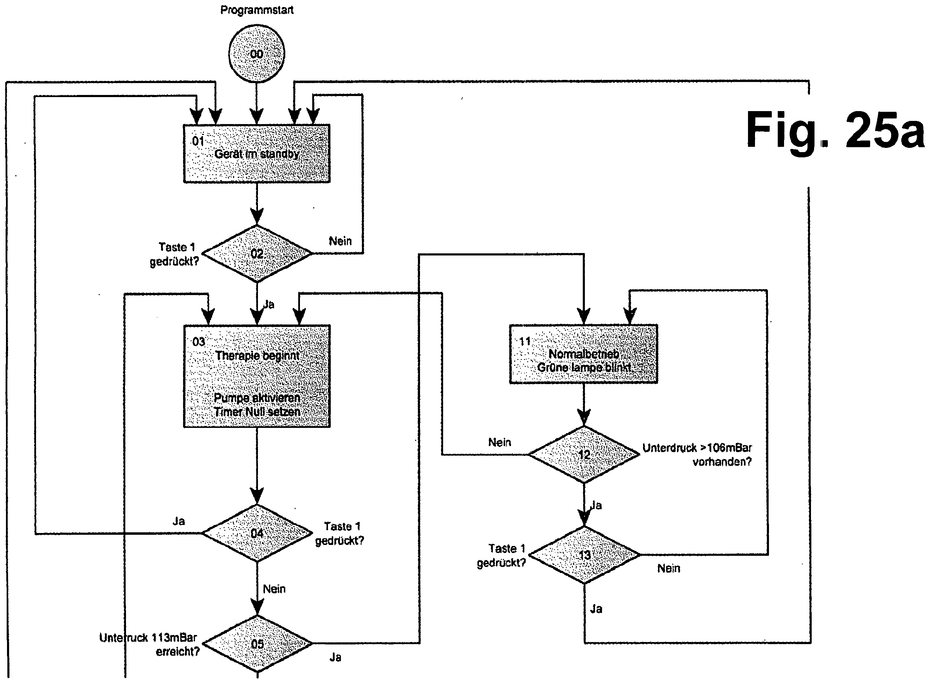

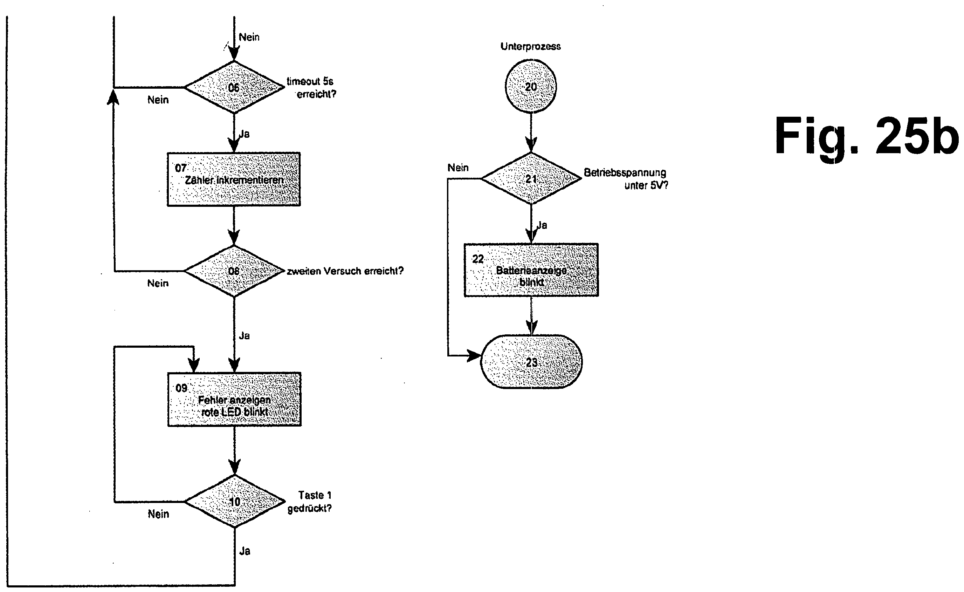

- Fig. 25: einen exemplarischen Betriebsmodus einer erfindungsgemäßen Vorrichtung, und

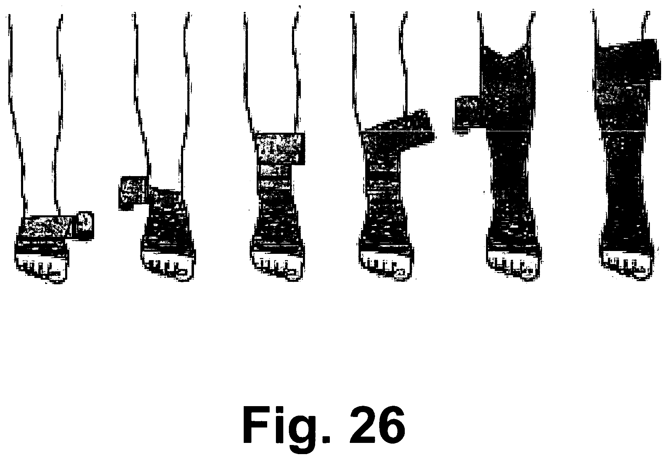

- Fig. 26: einen Kompressionsverband, in welchem die erfindungsgemäße Vorrichtung angewandt werden kann.

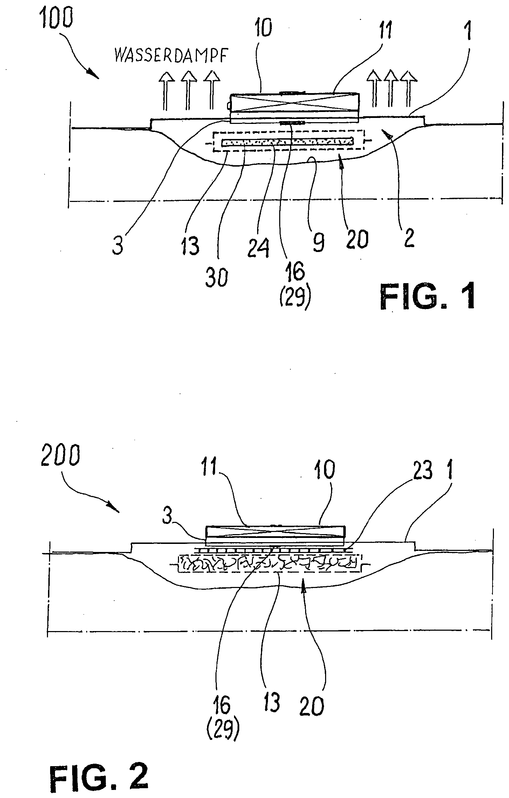

In Fig. 1 ist eine Wundpflegevorrichtung 100 dargestellt, bestehend aus einem flüssigkeitsundurchlässigen, jedoch wasserdampfdurchlässigen Wundabdeckungselement 1, einem umhüllten Absorptionskörper 20 und einer Einrichtung zur Erzeugung von atmosphärischem Unterdruck. Als Einrichtung zur Erzeugung von Unterdruck kommt eine Mikropumpe 11 zum Einsatz.

Das Wundabdeckungselement 1 ist in Form einer folienartigen und flexiblen Membrane angefertigt.

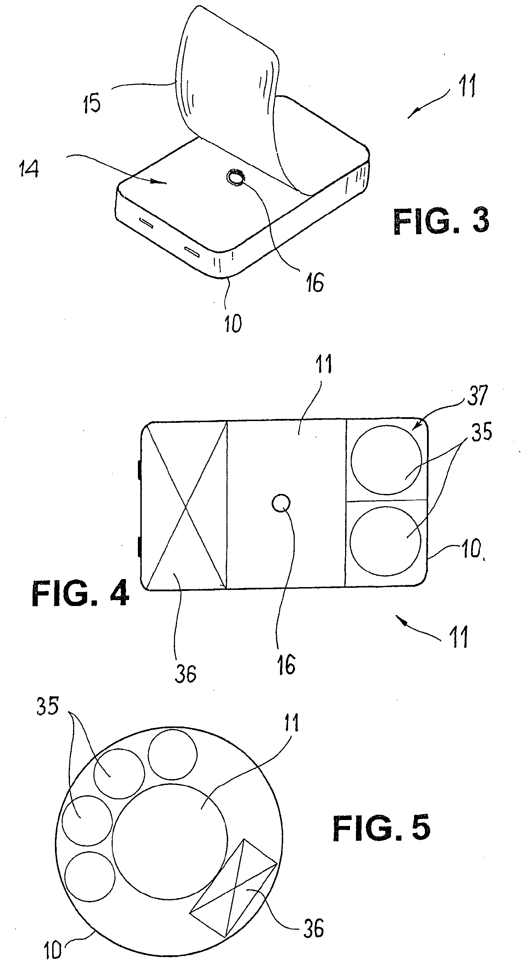

Die Mikropumpe 11 weist ein rechteckiges Flachgehäuse 10 auf, das mit einer Ansaugöffnung 16 (vgl. Fig. 3), im vorliegenden Fall mit Rückschlagventil 29, versehen ist.

Als Mikropumpe 11 wird eine piezoelektrische Membranpumpe verstanden, die einen geringen Energieverbrauch, kompakten Aufbau und sehr kleine Ausmaße aufweist, die den Einsatz der Mikropumpe in portablen Systemen ermöglichen. Insbesondere ist es möglich, die Mikropumpe 11 direkt am Wundabdeckungselement 1 anzuordnen, da sie ein sehr geringes Gewicht aufweist. Die Mikropumpe 11 samt Steuerung und optional mit Batterie (-n) ist in einem Flachgehäuse 10 untergebracht.

Gemäß Fig. 1 ist die Mikropumpe 11 (Hersteller: Bartels Mikrotechnik GmbH, Dortmund, Deutschland) auf der wundabgewandten Seite des Wundabdeckungselementes 1 angeordnet und dort mit dem Wundabdeckungselement 1 lösbar verklebt. So lässt sich nach Bedarf wieder verwenden, wenn Sicherheitsmaßnahmen, wie Sterilisation, vorgenommen werden.

Weiterhin ist der Fig. 1 ein Wundhohlraum 2 zu entnehmen, der von dem an die Haut des Patienten aufgeklebten Wundabdeckungselement 1 und einem Wundgrund 9 begrenzt ist. Der im Wundhohlraum 2 befindliche Absorptionskörper 20 besteht aus einem Abschnitt 30 einer vliesartigen Textilmatte, die mit einer superabsorbierenden Substanz 24 angereichert ist, und aus einer Hülle 13.

In Fig. 2 ist eine ähnliche Wundpflegevorrichtung (Bezugszeichen 200) dargestellt. Die Hülle weist jedoch anstelle der Textilmatte ein schaumartiges Innenleben auf. Zwischen dem Wundabdeckungselement 1 und dem Absorptionskörper 20 liegt ein Abstandskörper 23, der die Gase und Luft durchlässt.

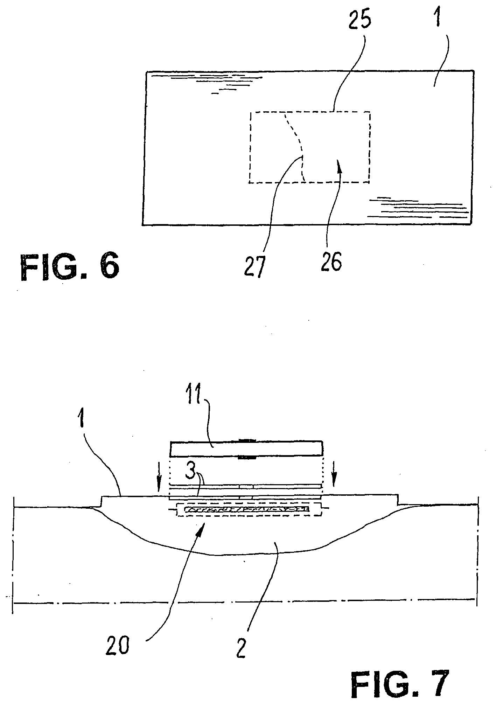

Die Anbringung der Mikropumpe 11 ist in Figuren 6 und 7 gezeigt. Das Wundabdeckungselement 1 weist eine der Flächengröße des Flachgehäuses 10 entsprechende Klebefläche 26 auf, die mit einem abziehbaren Schutzfolienelement 27 abgedeckt ist. Die Klebefläche 26 ist von einer Markierung 25 umgeben, die es erlaubt, das Flachgehäuse 10 der Mikropumpe an der gewünschten Stelle des Wundabdeckungselementes 1 (vgl. Fig. 7) anzubringen.

Die Mikropumpe 11 ist in Figuren 3, 4 und 5 in einer schematischen, vergrößerten Darstellung gezeigt. Zur lösbaren Anbringung des Flachgehäuses 10 am Wundabdeckungselement 1 wird zuerst ein Schutzfolienelement 15 abgezogen, das eine Klebefläche 14 sowie die Ansaugöffnung 16 freigibt.

Gemäß Fig. 4 ist das rechteckige Flachgehäuse 10 in drei Innenfächern aufgeteilt, die einen Steuermodul 36, die eigentliche Mikropumpe 11 und ein Batteriefach 37 aufweisen. Im Batteriefach 37 sind zwei Knopfzellen 35 untergebracht.

Die Fig. 5 zeigt ein rundes Flachgehäuse, an dem peripher vier Knopfzellen 35 sowie der Steuermodul 36 angeordnet sind.

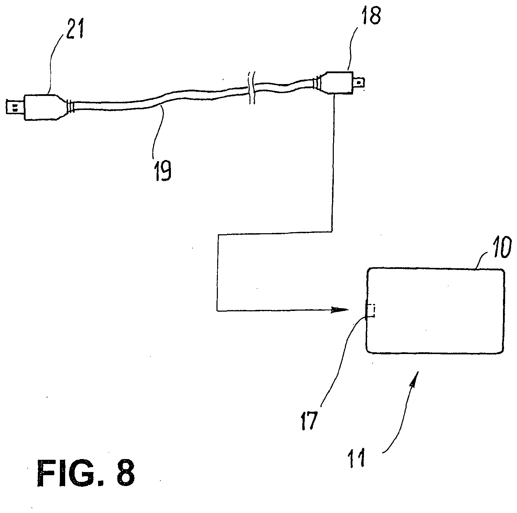

Wie die Fig. 8 zeigt, ist das Flachgehäuse 10 der Mikropumpe 11 mit einer Mini-USB-Anschlussbüchse 17 ausgestattet, in die ein Mini-USB-Anschluss 18 steckbar ist, der wiederum über ein Kabel 19 mit einem USB-Anschluss 21 für einen PC oder mit einem Stecker für Zigarettenanzünder verbunden ist. Das Anschluss-Prinzip ähnelt dem des handelsüblichen Navigationsgerätes. Dabei ist vorteilhaft, dass der Anschluss an den PC eine sofortige Auswertung erlaubt - z. B. Analyse des Wundexsudats - wenn die Mikropumpe mit einem Meßsensor (nicht dargestellt) ausgestattet ist.

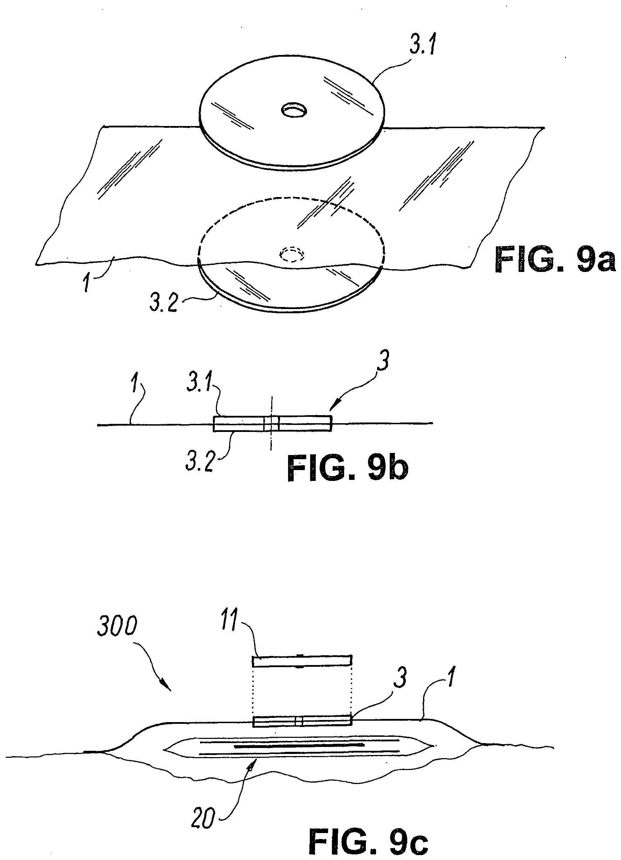

Eine erfindungsgemäße Wundpflegevorrichtung (Bezugszeichen 300) ist in Fig. 9c dargestellt. Die Mikropumpe 11 steht schlauchlos, jedoch über einen Flachadapter 3 mit dem Wundhohlraum 2 in Kontakt, so dass nicht direkt mit dem Wundabdeckungselement 1 verbunden ist (vgl. Figuren 9a und 9b). Der Flachadapter 3 besteht aus zwei zueinander deckungsgleichen Tellern 3.1, 3.2, die von "oben" und "unten" an dem Wundabdeckungselement 1 anbringbar sind. Zumindest einer der beiden Teller 3.1, 3.2 des Flachadapters kann aus Kunststoff, Metall oder Magnetfolie bestehen.

Die Teller 3.1, 3.2 können miteinander über das Wundabdeckungs-element 1 rastend verbunden oder verklebt sein. Hinsichtlich der Dichtigkeit wird jedoch eine Klebeverbindung bevorzugt.

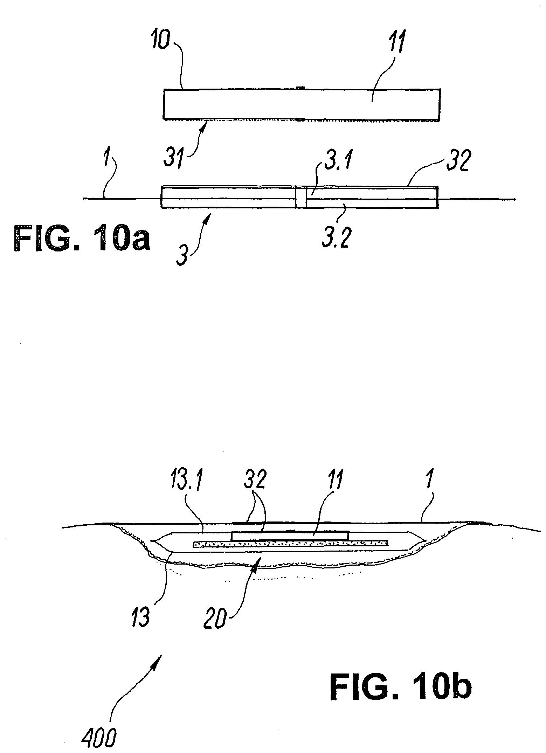

Wenn der obere Teller 3.1 magnetisch ist, kann das Flachgehäuse 10 der Mikropumpe, das mit einer sehr dünnen Magnetfolie oder mit Magnetpulver beschichtet ist, auf den Flachadapter 3 einfach gelegt werden. In vorliegendem Fall (vgl. Fig. 10a) ist das Flachgehäuse 10 auf seiner dem Wundabdeckungselement 1 zugewandten Seite mit einer Magnetpulverschicht 31 und der obere Teller 3.1 mit einer Magnetfolie 32 versehen.

Von Vorteil ist, dass der Flachadapter 3 die Lagestabilität der abnehmbaren Mikropumpe 11 verbessert und derer Montage an dem Wundabdeckungselement 1 erleichtert.

Eine andere Ausführungsform der Magnetverbindung ist der Fig. 10b zu entnehmen. Die Fig. 10b zeigt eine Wundpflegevorrichtung 400, bei der die Mikropumpe 11 unterhalb eines oberen, dem Wundabdeckungs-element 1 zugewandten Hüllenabschnittes 13.1 der Hülle 13 des Absorptionskörpers 20 liegt. So wird erreicht, dass die Mikropumpe 11 wenigstens in Anfangsphase der Wundtherapie unterhalb des Hautniveau an der Wunde angeordnet sein kann. Unterhalb des Absorptionskörpers 20 ist noch ein schmiegsamer Wunddistanzgitter 33 (Produkt SORBION PLUS, Hersteller: SORBION AG, Senden, Deutschland) angeordnet. Optional kann anstelle des Wunddistanzgitters 33 ein mit Silber oder Kupfer angereichter, maschenartiger Textilabschnitt zum Einsatz kommen.

Die obere Flachseite des Flachgehäuses der Mikropumpe 11 sowie das Wundabdeckungselement 1 sind jeweils mit der Magnetfolie 32 versehen. Anstelle der Magnetfolie kann eine Rastverbindung (nicht dargestellt) zum Einsatz kommen.

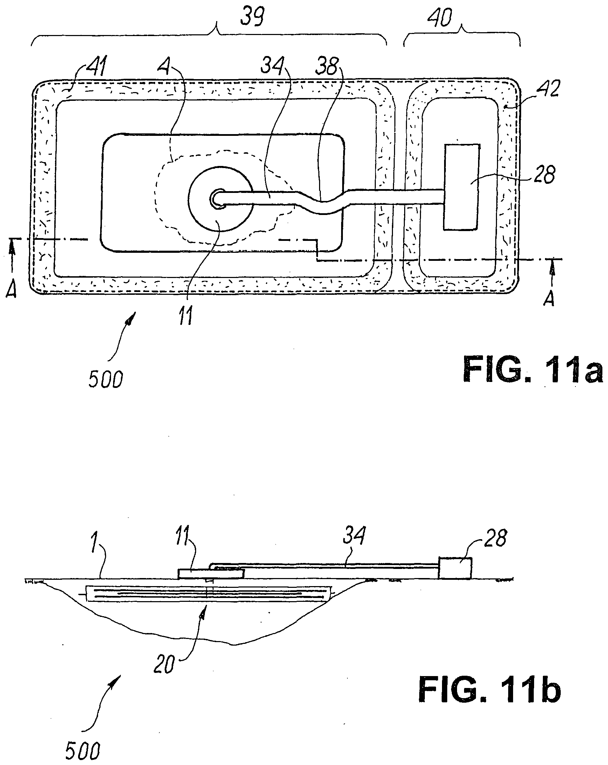

Eine in Figuren 11a und 11b gezeigte Wundpflegevorrichtung 500 besteht aus dem Wundabdeckungselement 1, der Mikropumpe 11, einer Akku-Batterie 28 und einem dazwischen liegenden und diese elektrisch verbindenden Kabel 34. Das Kabel 34 weist einen bogenförmigen Kompensationsabschnitt 38 auf, mit dem sich die Längenveränderungen bei der Volumenzunahme des Absorptionskörpers 20 ausgleichen lassen. Das Wundabdeckungselement 1 ist wiederum in einen Wundbehandlungsbereich 39 und einem Batteriebereich 40 aufgeteilt, wobei die beiden Bereiche voneinander mit peripher umlaufenden Klebeflächen 41,42 getrennt sind. Auf dieser Weise kann an einem Wundabdeckungselement 1 sowohl die Mikropumpe 11 als auch die Akku-Batterie 28 vormontiert sein. Die Akku-Batterie 28 liegt jedoch außerhalb des Wundbehandlungsbereichs 39 und insbesondere außerhalb einer Wundkontur 4.

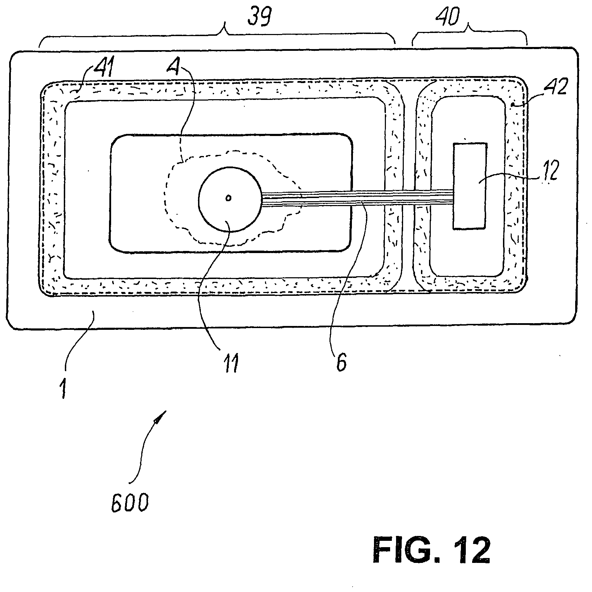

Dasselbe Konstruktionsprinzip gilt für eine Wundpflegevorrichtung 600 gemäß Fig. 12. Gleiche Teile sind mit gleichen Bezugszeichen bezeichnet. Eine elektrische Verbindung der Mikropumpe 11 mit einer Lithium-Ionen-Batterie 12 sichert eine folienartige gedruckte Schaltung 6.

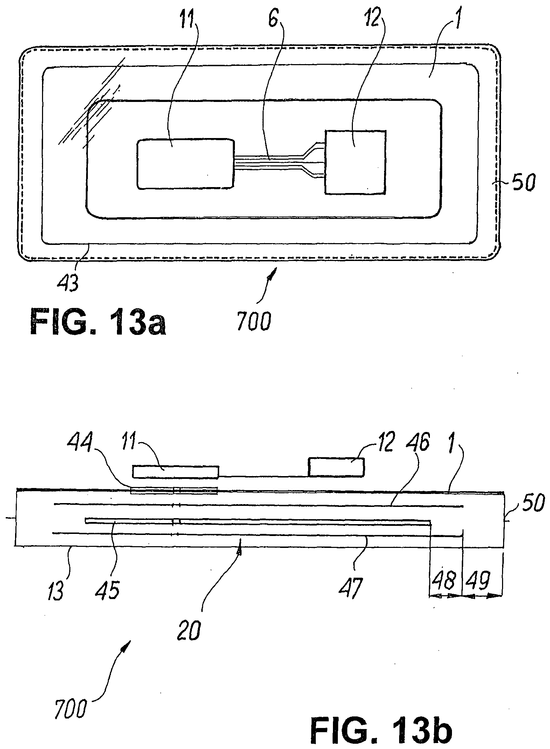

Die Figuren 13a und 13b zeigen eine Wundpflegevorrichtung 700, die eine Weiterentwicklung der in Fig. 12 gezeigten Wundpflegevorrich-tung 600 darstellt. Die Mikropumpe 11 und die Lithium-Ionen-Batterie 12 samt gedruckter Schaltung 6 sind innerhalb eines Umrisses 43 des Absorptionskörpers 20 angeordnet. Die Mikropumpe 11 ist über einen rechteckigen, in das Wundabdeckungselement 1 eingebauten Flachadapter 44 einrastend verbunden.
Wie die Fig. 13 b zeigt, weist der Absorptionskörper 20 die besagte Hülle 13 auf, innerhalb deren zwei superabsorbierende Textil-abschnitte 46, 47 und eine dazwischen liegende superabsorbierende, flächenmäßig kleinere Zellstoff-Matte 45 angeordnet sind. Die Flächenunterschiede zwischen den Textilmatten sowie einer umlaufenden Naht 50 der Hülle ergeben gewünschte Expansionsräume 48, 49.

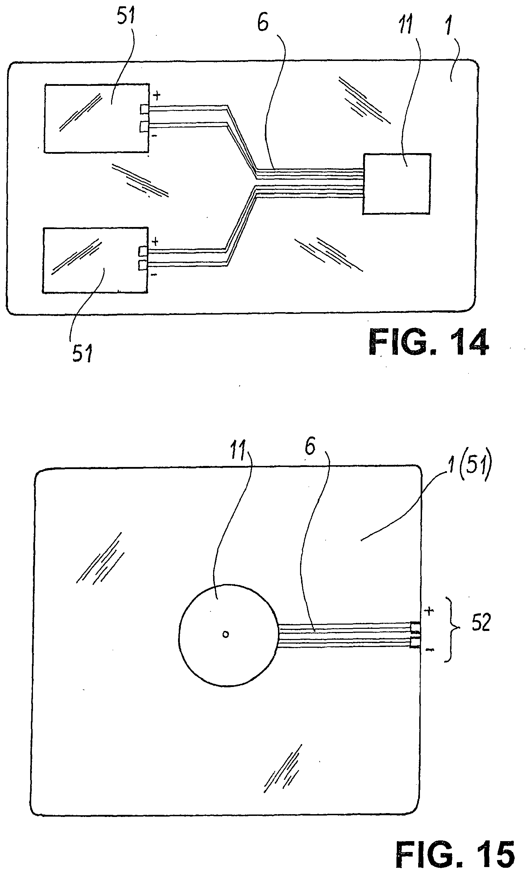

Ein anderes, in Fig. 14 dargestelltes Wundabdeckungselement 1 weist zwei elastische, folienartige Flachbatterien 51 auf, die auf das Wundabdeckungselement 1 aufgeklebt sind und über die gedruckte Schaltung 6 elektrisch mit der Mikropumpe 11 verbunden sind. Die Flachbatterie stellt eine Neuentwicklung der elektrisch leitenden Polymerfolie dar, die in der Fachpresse als "Papierbatterie" bezeichnet wird.

Das in Fig. 15 gezeigte Wundabdeckungselement 1 von einem Format 10cm x 10cm besteht aus einer elektrisch leitenden, dünnen, biegsamen Polymerfolie. Aus dem Grund fungiert das Wundabdeckungselement 1 zu einer Flachbatterie 51. Über die gedruckte Schaltung 6 ist die zentral an der Flachbatterie 51 angeordnete Mikropumpe 11 mit Polen 52 (plus, minus) verbunden, die in die Polymerfolie eingebaut sind.

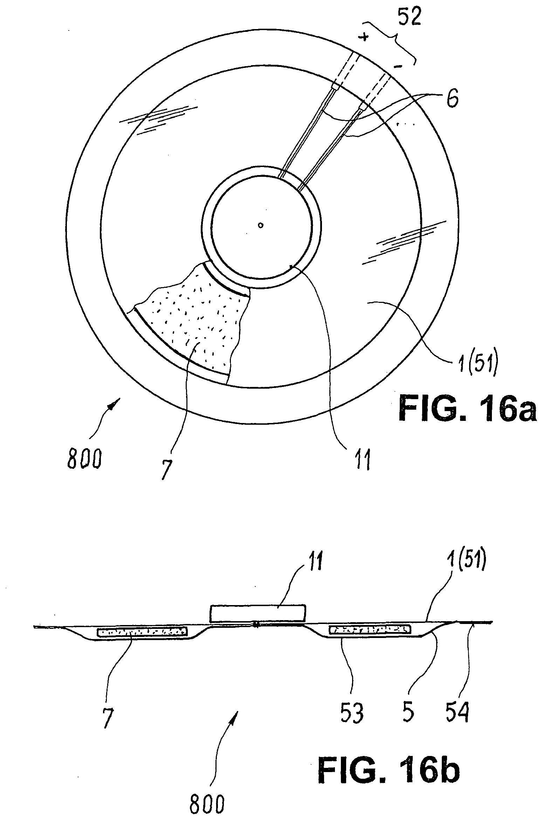

Die Figuren 16a und 16b zeigen eine flache, kreisrunde Wundpflegevorrichtung 800, die einen ringförmigen, umhüllten Absorptionskörper 7, die zentral angeordnete Mikropumpe 11 und die gedruckte Schaltung 6 aufweist. Das Wundabdeckungselement 1 ist zugleich eine kreisrunde Flachbatterie 51. Die Mikropumpe 11 ist über die besagte gedruckte Schaltung 6 an die Pole 52 elektrisch angeschlossen. Das Wundabdeckungselement 1 bildet zusammen mit einem ringförmigen, wundzugewandten Folienabschnitt 53 eine Hülle 5, die eine periphere Klebefläche 54 aufweist.

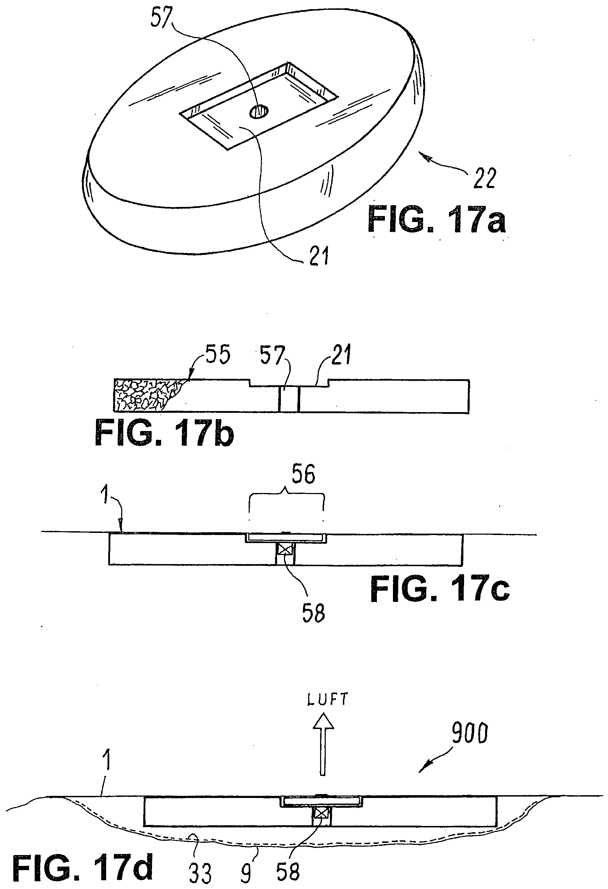

Eine weitere Wundpflegevorrichtung (Bezugszahl 900) ist der Fig. 17d zu entnehmen. Die Wundpflegevorrichtung 900 weist einen schaumartigen, aus Polyurethan gefertigten Absorptionskörper 22 auf, in dessen Mitte ein rechteckiger Sitz 21 (vgl. Figuren 17b und 17c) zur Aufnahme der Mikropumpe 11 derart ausgespart ist, dass die obere Flachseite der Mikropumpe 11 mit einer Oberfläche 55 des schaumartigen Absorptionskörpers in Flucht liegt. Ferner ist am Wundabdeckungselement 1 ein Release-Kleber-Bereich 56 vorgesehen, der sich ungefähr mit dem Sitz 21 deckt. Der übrige Bereich des Wundabdeckungselementes 1 ist mit dem Absorptionskörper 22 nicht verbunden. In einer durchgehenden Öffnung 57 des Absorptionskörpers 22 ist ein Rückschlagventil 58 platziert. Zwischen dem Absorptions-körper 22 und dem Wundgrund 9 liegt der Wunddistanzgitter 33.

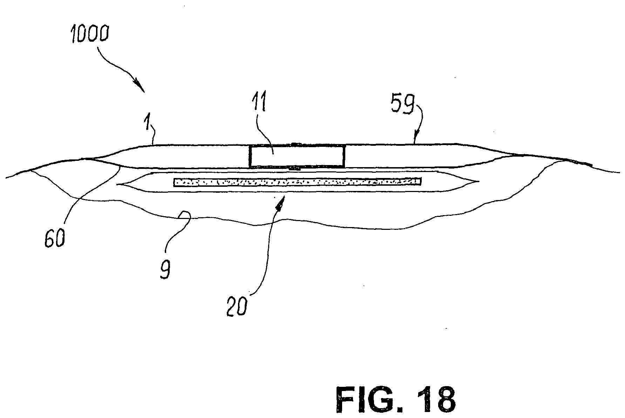

In Fig. 18 ist eine Wundpflegevorrichtung 1000 dargestellt, bei der die Mikropumpe 11 innerhalb einer folienartigen Hülle 59 platziert ist, die von dem Wundabdeckungselement 1 und einem "unteren", wundzugewandten Hüllenabschnitt 60 gebildet ist. Der untere Hüllenabschnitt 60 ist gas- und flüssigkeitsdurchlässig, dagegen das Wundabdeckungselement 1 nur wasserdampfdurchlässig. Unterhalb der Hülle 59 liegt der Absorptionskörper 20.

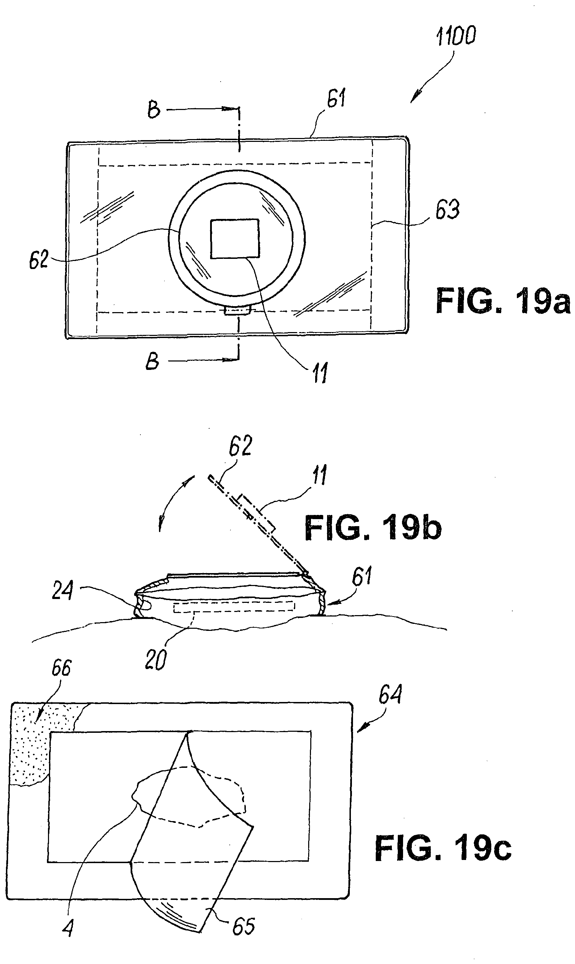

Die Figuren 19a bis 19c zeigen eine beutelartige Wundpflegevorrichtung 1100, aufweisend einen Beutel 61 in der Art eines bekannten Drainagekollektors mit einer schwenkbaren (vgl. Fig. 19b) Fenster-klappe 62. Der Beutel 61 ist an seinem Umfang 63 zusammengefaltet. Die Mikropumpe 11 ist mitten an der Fensterklappe 62 angeordnet und mit einer nicht dargestellten Batterie elektrisch verbunden. Die Batteriefunktion kann das transparente Folienelement (Flachbatterie) der Fensterklappe 62 übernehmen (nicht gezeigt).

Die Innenfläche des Beutels 61 ist mit der superabsorbierenden Substanz 24 (vgl. Fig. 19b) verkleidet bzw. beschichtet. Die superabsorbierende Substanz 24 kann gelförmig oder in ein Textilmaterial eingestreut sein.

Gemäß Fig. 19c weist der Beutel 61 einen folienartigen, klebebe-schichteten Boden 64 auf, der der Wundkontur 9 entsprechend ausgespart wird, so dass ein mittlerer Bodenabschnitt 65 vom Boden abgezogen und der Boden 64 mit seiner peripheren Klebefläche 66 auf die Haut des Patienten aufgeklebt werden kann. Innerhalb des Beutels 61 befindet sich der über die schwenkbare Fensterklappe 62 dort eingelegte, herausnehmbare Absorptionskörper 20. Eine weitere Ausführungsform (nicht dargestellt) sieht einen Boden vor, der aus dem besagten Wunddistanzgitter 33 (Produkt SORBION PLUS, Hersteller: SORBION AG) gefertigt und mit einer umlaufenden Klebefläche versehen ist.

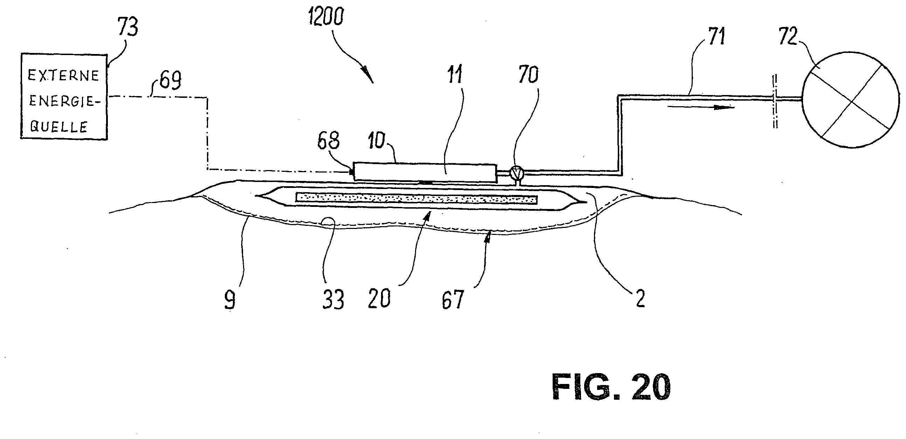

Eine besonders vorteilhafte Wundpflegevorrichtung 1200 ist der Figur 20 zu entnehmen. Die Wundpflegevorrichtung 1200 ähnelt der in Fig. 1 dargestellten Wundpflegevorrichtung 100. Das Flachgehäuse 10 der Mikropumpe 11 ist auf der wundabgewandten Seite des Wundabdeckungs-elementes 1 angeordnet und dort mit dem Wundabdeckungselement 1 lösbar verklebt. Unterhalb des umhüllten Absorptionskörpers 20 liegt der Wunddistanzgitter 33, der mit seiner glatten Oberfläche 67 auf den Wundgrund 9 gerichtet ist. Auf den Wundgrund 9 wird zuerst der Wunddistanzgitter 33 und danach der Absorptionskörper 20 (Produkte: SORBION PLUS und SORBION SACHET, Hersteller: SORBION, Senden, Deutschland) gelegt. Das folienartige Wundabdeckungselement 1 wird dicht um die Wunde an die Haut des Patienten angeklebt.

Mit dem Wundabdeckungselement 1 wird dann die Mikropumpe 11 lösbar verklebt. Die Mikropumpe 11 beinhaltet die in Fig. 4 gezeigten Batterien. Allerdings ist die Mikropumpe 11 zusätzlich über einen koaxialen Anschluss 68 und Kabel 69 an eine externe Energiequelle 73 angeschlossen. Die Energiequelle kann ein Akkumulator oder ein Stromnetz sein.

Die Mikropumpe 11 ist über ein Dreiwegeventil 70 und Vakuumleitung 71 an eine externe Vakuumpumpe 72 angeschlossen. Die Mikropumpe 11 ist zuerst durch das Dreiwegeventil 70 blockiert. Die Mikropumpe 11 arbeitet nicht. Durch das Betätigen der externen Vakuumpumpe 72 wird die Luft aus dem Wundhohlraum 2 nahezu vollständig evakuiert. Durch Verstellung des Dreiwegeventils 70 wird die externe Vakuumpumpe 72 automatisch ausgeschaltet. In diesem Moment übernimmt die Mikropumpe 11 die Saugfunktion. Der durch die externe Vakuumpumpe 72 erreichte Unterdruck wird durch die Mikropumpe 11 aufrechterhalten. Das System kann mit einer einprogrammierten Intervallschaltung ausgestattet sein, die die Mikropumpe 11 nach Bedarf in Gang setzt. Diese Intervallschaltung kann mit dem Steuerungsmodul verbunden oder Teil des Steuerungsmoduls sein.

Der Steuerungsmodul kann ferngesteuert sein (beispielsweise vom Patienten oder Pflegepersonal). Die Fernsteuerung kann auch über herkömmliche Fernbedienung betätigbar sein.

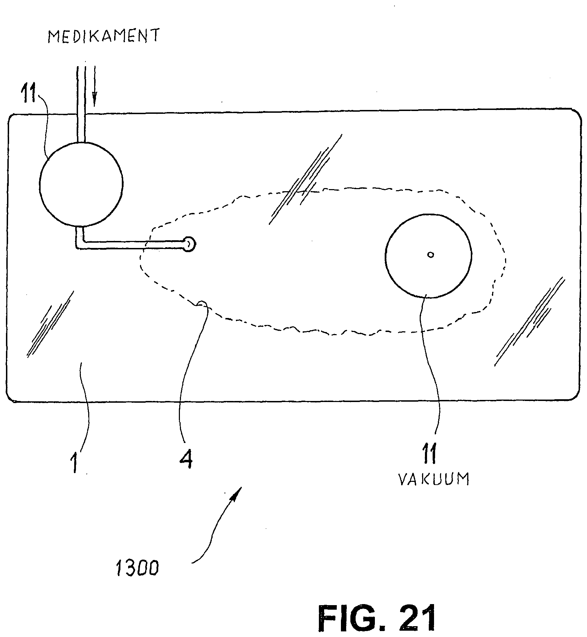

In Fig. 21 ist eine besondere Ausführungsform der Wundpflegevorrichtung (Bezugszeichen 1300) gezeigt, die von ihrer Funktion her dem erfindungsgemäßen Prinzip abweicht. Der Absorptionskörper ist wegen der Klarheit der Zeichnung weggelassen. Am Wundabdeckungselement 1 sind zwei Mikropumpen 11 angeordnet, von denen die eine die Funktion der Vakuumpumpe und die andere die Funktion einer Dosierpumpe für Medikamente bzw. eine Spülfunktion erfüllt. Die Mikropumpen 11 sind reversibel, d. h. die Ventile an der Mikropumpe geben vor, in welcher Richtung die Fluide aus der Pumpe gedrückt werden.

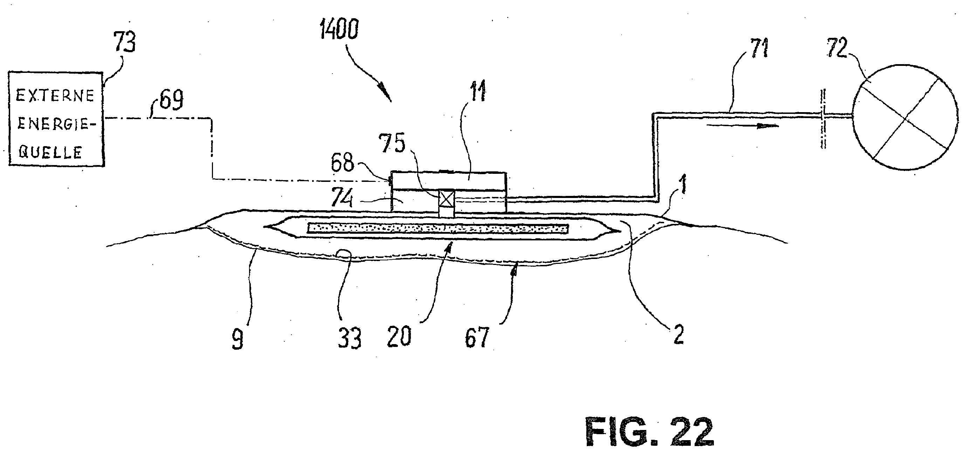

Die Fig. 22 zeigt eine Wundpflegevorrichtung 1400, bei der am Wundabdeckungselement 1 ein Saugkopf 74 befestigt ist, an dem wiederum die Mikropumpe 11 sitzt. Die Mikropumpe 11 ist also nicht an der Wundabdeckung, sondern am Saugkopf 74 angeordnet. Die Mikropumpe 11 kann über eine Rast- oder Klickverbindung, über eine Klebeverbindung oder magnetisch an dem Saugkopf 74 lösbar befestigt sein.

Der Saugkopf 74 ist mit einem Dreiwegeventil 75 versehen, an das über die 71 Vakuumleitung die externe Vakuumpumpe 72 angeschlossen ist. Der schematisch dargestellte Saugkopf 74 kann stark abgeflacht sein. Sonst zeichnet sich die Wundpflegevorrichtung 1400 durch gleichen Aufbau und durch gleiche Funktion aus, die bereits bei der Fig. 20 beschrieben worden sind.

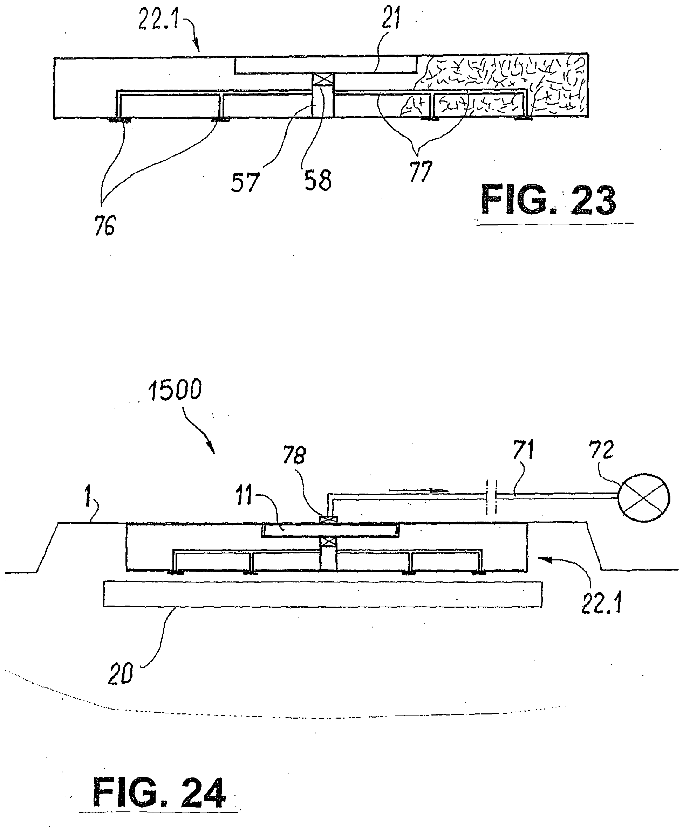

In Fig. 23 ist ein aus weichem Polyurethan-Schaum gefertigter Absorptionskörper 22.1 dargestellt, der dem Absorptionskörper 22 (vgl. Fig. 17) ähnlich ist. Der Unterschied zwischen den beiden Absorptionskörpern 22 und 22.1 besteht darin, dass der letztere mit mehreren, an dessen Unterseite verteilten, kleinen Saugköpfen 76 bestückt ist, welche jeweils über eine Leitung 77 an die am Schaum eingearbeitete Öffnung 57 mit Rückschlagventil 58 angeschlossen sind. Die "Mini-Saugköpfe" tragen zu einer verbesserten Druckverteilung bei.

Der Absorptionskörper 22.1 ist gemäß Fig. 24 Teil einer Wundpflege-vorrichtung 1500. Die Wundpflegevorrichtung 1500 enthält außerdem das besagte Wundabdeckungselement 1, die in den Sitz 21 des Absorptionskörpers 22.1 platzierte Mikropumpe 11 und den dem Wundgrund (nicht dargestellt) zugewandten Absorptionskörper 20. Der schaumartige Absorptionskörper 22.1 weist eine wesentlich kleinere Saugkraft als die des Absorptionskörpers 20 auf. Außerdem erfüllt er die Rolle eines Puffers zwischen der Mikropumpe und des unteren Absorptionskörpers 20. Zwischen den beiden Absorptionskörpern 22 und 22.1 kann noch eine nicht gezeigte, kleinporige Membrane liegen.

Über ein an der Mikropumpe angeordnetes Dreiwegeventil 78 und die Vakuumleitung 71 ist die Mikropumpe 11 an die externe Vakuumpumpe 72 angeschlossen. Die Mikropumpe 11 kann über wenigstens eine Knopfzelle verfügen oder/und an eine externe Energiequelle (vgl. Fig. 20) angeschlossen sein.

### Bezugszeichenliste:

- 1: Wundabdeckungselement
- 2: Wundhohlraum
- 3: Flachdapter
- 4: Wundkontur
- 5: Hülle
- 6: gedruckte Schaltung
- 7: Absorptionskörper
- 9: Wundgrund
- 10: Flachgehäuse
- 11: Mikropumpe
- 12: Lithium-Ionen-Batterie
- 13: Hülle
- 13.1: Hüllenabschnitt
- 14: Klebefläche
- 15: Schutzfolienelement
- 16: Ansaugöffnung
- 17: Mini-USB-Anschlussbüchse
- 18: Mini-USB-Anschluss
- 19: Kabel
- 20: Absorptionskörper
- 21: Sitz
- 22: Absorptionskörper
- 22.1: Absorptionskörper
- 23: Abstandskörper
- 24: superabsorbierende Substanz
- 25: Markierung
- 26: Klebefläche
- 27: Schutzfolienelement
- 28: Akku-Batterie
- 29: Rückschlagventil
- 30: Abschnitt (Textil)
- 31: Magnetpulverschicht
- 32: Magnetfolie
- 33: Wunddistanzgitter
- 34: Kabel
- 35: Knopfzelle
- 36: Steuermodul
- 37: Batteriefach
- 38: Kompensationsabschnitt
- 39: Wundbehandlungsbereich
- 40: Batteriebereich 40
- 41, 42: Klebefläche
- 43: Umriss
- 44: Flachadapter
- 45: Zellstoff-Matte
- 46, 47: Textilabschnitt
- 48, 49: Expansionsraum
- 50: Naht
- 51: Flachbatterie ("Papierbatterie")
- 52: Pol
- 53: Folienabschnitt
- 54: Klebefläche
- 55: Oberfläche
- 56: Release-Kleber-Bereich
- 57: Öffnung
- 58: Rückschlagventil
- 59: Hülle
- 60: Hüllenabschnitt
- 61: Beutel
- 62: Fensterklappe
- 63: Umfang
- 64: Boden
- 65: Bodenabschnitt
- 66: periphere Klebefläche
- 67: Oberfläche
- 68: Anschluss
- 69: Kabel
- 70: Dreiwegeventil
- 71: Vakuumleitung
- 72: externe Vakuumpumpe
- 73: externe Energiequelle
- 74: Saugkopf
- 75: Dreiwegeventil
- 76: Saugkopf
- 77: Leitung
- 78: Dreiwegeventil
- 79: Öffnung
- 100; 200; 300: Wundpflegevorrichtung
- 400; 500; 600: Wundpflegevorrichtung
- 700; 800; 900: Wundpflegevorrichtung
- 1000; 1100: Wundpflegevorrichtung
- 1200; 1300: Wundpflegevorrichtung
- 1400; 1500: Wundpflegevorrichtung

## Patentansprüche

1. Wundpflegevorrichtung zur Behandlung von Wunden mittels atmosphärischem Unterdruck im Wundbereich, aufweisend:
- wenigstens ein Wundabdeckungselement (1), sowie
- wenigstens eine Mikropumpe, die schlauchlos über einen Flachadapter (3) mit einem Wundhohlraum (2) in Verbindung steht, wobei der Flachadapter (3) aus zwei zueinander deckungsgleichen Tellern (3.1, 3.2) besteht, die von oben und von unten an dem Wundabdeckungselement (1) angebracht sind,
wobei die Vorrichtung ferner wenigstens einen im Wesentlichen flachen einen Wundexsudate aufsaugenden Absorptionskörper aus Absorptionsmaterial aufweist, der ein aufsaugendes Vlies mit darin verteilten superabsorbierenden Polymeren aufweist,
und wobei das Vlies Zellulosefasern aufweist.

2. Wundpflegevorrichtung gemäß Anspruch 1, wobei die Vorrichtung ferner wenigstens ein Wundexsudate aufnehmendes Reservoir aufweist.

3. Wundpflegevorrichtung gemäß einem der vorhergehenden Ansprüche, wobei die Vorrichtung ferner eine wundzugewandte flüssigkeitsdurchlässige Wundkontaktschicht aufweist.

4. Wundpflegevorrichtung gemäß einem der vorhergehenden Ansprüche, wobei zwischen der Mikropumpe und dem Wundsekrete aufsaugenden Absorptionskörper eine Barriere angeordnet ist, die keine Flüssigkeiten passieren lässt.

5. Wundpflegevorrichtung gemäß einem der vorhergehenden Ansprüche, wobei die Mikropumpe auf der wundabgewandten Seite des Wundabdeckungselements angeordnet ist.

6. Wundpflegevorrichtung gemäß einem der vorhergehenden Ansprüche, wobei die Mikropumpe zwei oder mehr parallel oder in Reihe angeordnete Pumpen aufweist.

7. Wundpflegevorrichtung gemäß einem der vorhergehenden Ansprüche, wobei die Vorrichtung ferner mindestens eine Batterie (12.1, 12.2) umfasst, die bevorzugt ausgewählt ist aus der Gruppe enthaltend
• Herkömmliche Einwegbatterien
• Herkömmliche wiederaufladbare Batterien
• Arrays von Kleinbatterien, bevorzugt "Knopfzellen"
• Flexible, biegsame wiederaufladbare Akkumulatoren.

8. Wundpflegevorrichtung gemäß einem der vorhergehenden Ansprüche, wobei die Vorrichtung eine Anschlussstelle für eine externe Stromversorgung aufweist.

9. Wundpflegevorrichtung gemäß einem der vorhergehenden Ansprüche, wobei zwischen der Mikropumpe und dem Wundexsudate aufsaugenden Absorptionskörper eine Kupplung, ein Absperrventil und/oder ein Dreiwegehahn angeordnet ist.

10. Wundpflegevorrichtung gemäß einem der vorhergehenden Ansprüche, wobei die Vorrichtung ferner einen Sensor für mindestens einen physiologischen und/oder pathologischen Parameter des Wundexsudats aufweist.

## Claims

1. Wound care device for treating wounds by means of negative atmospheric pressure in the wound area, having:
- at least one wound covering element (1), which delimits a wound cavity (2) when used with a wound bed (9), and
- at least one micropump, which is connected tubelessly via a flat adapter (3) to the wound cavity (2), wherein the flat adapter (3) consists of two plates (3.1, 3.2) congruent with one another, which are applied to the wound covering element (1) from above and from below,
wherein the device further comprises at least one substantially flat absorption body of absorption material, which body absorbs a wound exudate and comprises an absorbent nonwoven with superabsorbent polymers distributed therein,
and wherein the nonwoven has cellulose fibres.

2. Wound care device according to claim 1, wherein the device further has at least one reservoir receiving a wound exudate.

3. Wound care device according to one of the preceding claims, wherein the device further has a liquid-permeable wound contact layer facing the wound.

4. Wound care device according to any one of the preceding claims, wherein arranged between the micropump and the absorption body absorbing wound secretions is a barrier, which does not allow any liquids to pass.

5. Wound care device according to any one of the preceding claims, wherein the micropump is arranged on the side of the wound covering element facing away from the wound.

6. Wound care device according to any one of the preceding claims, wherein the micropump has two or more pumps arranged in parallel or in series.

7. Wound care device according to any one of the preceding claims, wherein the device further comprises at least one battery (12.1, 12.2), which is preferably selected from the group containing
• conventional disposable batteries
• conventional rechargeable batteries
• arrays of small batteries, preferably "button cells"
• flexible, bending rechargeable accumulator batteries.

8. Wound care device according to any one of the preceding claims, wherein the device has a connection point for an external power supply.

9. Wound care device according to any one of the preceding claims, wherein arranged between the micropump and the absorption body absorbing wound exudate is a coupling, a shut-off valve and/or a three-way valve.

10. Wound care device according to any one of the preceding claims, wherein the device further has a sensor for at least one physiological and/or pathological parameter of the wound exudate.

## Revendications

1. Dispositif de soin de plaies destiné à traiter des plaies au moyen d'une dépression atmosphérique dans la zone de la plaie, comportant :
- au moins un élément de recouvrement de plaie (1), qui, lors de l'utilisation, limite une cavité de plaie (2) avec un lit de plaie (9), ainsi que
- au moins une micropompe, qui est en liaison avec la cavité de plaie (2) sans tuyau par le biais d'un adaptateur plat (3), l'adaptateur plat (3) se composant de deux disques (3.1, 32) coïncidant l'un avec l'autre, qui sont appliqués par le haut et par le bas sur l'élément de recouvrement de plaie (1),
le dispositif comportant en outre au moins un corps d'absorption sensiblement plat et absorbant un exsudat de la plaie, constitué d'un matériau d'absorption qui comporte un nontissé absorbant doté de polymères superabsorbants répartis dans celui-ci,
et le nontissé comportant des fibres de cellulose.

2. Dispositif de soin de plaies selon la revendication 1, le dispositif comportant en outre au moins un réservoir absorbant des exsudats de la plaie.

3. Dispositif de soin de plaies selon l'une des revendications précédentes, le dispositif comportant en outre une couche de contact avec la plaie perméable aux liquides et orientée vers la plaie.

4. Dispositif de soin de plaies selon l'une des revendications précédentes, dans lequel une barrière est disposée entre la micropompe et le corps d'absorption absorbant les sécrétions de la plaies, ladite barrière ne laissant passer aucun liquide.

5. Dispositif de soin de plaies selon l'une des revendications précédentes, dans lequel la micropompe est disposée sur le côté orienté dans la direction opposée à la plaie de l'élément de recouvrement de plaie.

6. Dispositif de soin de plaies selon l'une des revendications précédentes, dans lequel la micropompe comporte deux pompes ou plus disposées en parallèle ou en série.

7. Dispositif de soin de plaies selon l'une des revendications précédentes, le dispositif comprenant en outre au moins une pile ou une batterie (12.1, 12.2), qui est de préférence sélectionnée dans le groupe contenant
• piles conventionnelles
• accumulateurs conventionnels
• ensembles de petites piles, de préférence « piles boutons »
• accumulateurs rechargeables souples flexibles.

8. Dispositif de soin de plaies selon l'une des revendications précédentes, le dispositif comportant un point de raccordement pour une alimentation en courant externe.

9. Dispositif de soin de plaies selon l'une des revendications précédentes, dans lequel un élément de couplage, une soupape d'arrêt et/ou un robinet à trois voies sont disposés entre la micropompe et le corps d'absorption absorbant les exsudats de la plaie.

10. Dispositif de soin de plaies selon l'une des revendications précédentes, le dispositif comportant en outre un capteur pour au moins un paramètre physiologique et/ou pathologique de l'exsudat de la plaie.
